# EUROPEAN PATENT SPECIFICATION

(11) **EP 3 461 890 B1**
(45) Date of publication and mention of the grant of the patent: **24.09.2025**
(21) Application number: 18204608.6
(22) Date of filing: 31.03.2014
(51) Int. Cl.: C12N 9/10, C12P 19/18, C12N 15/70, C12P 19/04

(54) **TOTAL FERMENTATION OF OLIGOSACCHARIDES**
VOLLSTÄNDIGE FERMENTATION VON OLIGOSACCHARIDEN
FERMENTATION TOTALE D'OLIGOSACCHARIDES

(43) Date of publication of application: 03.04.2019
(62) Divisional of application: 14162869.3
(73) Proprietor: Chr. Hansen HMO GmbH, 53619 Rheinbreitbach (DE)
(72) Inventor: Jennewein, Stefan, 53604 Bad Honnef (DE)
(74) Representative: NVS EPO Representatives

(56) References cited:
- WO-A1-2010/142305
- WO-A2-2006/034225
- WO-A2-2012/007481
- US-A1- 2002 042 369
- US-A1- 2009 082 307
- PRIEM BERNARD ET AL: "A new fermentation process allows large-scale production of human milk oligosaccharides by metabolically engineered bacteria", GLYCOBIOLOGY, OXFORD UNIVERSITY PRESS, US, vol. 12, no. 4, 1 April 2002 (2002-04-01), pages 235 - 240, XP002380632, ISSN: 0959-6658, DOI: 10.1093/GLYCOB/12.4.235
- RUFFING ANNE ET AL: "Metabolic engineering of microbes for oligosaccharide and polysaccharide synthesis", MICROBIAL CELL FACTORIES, BIOMED CENTRAL, LONDON, NL, vol. 5, no. 1, 21 July 2006 (2006-07-21), pages 25, XP021017786, ISSN: 1475-2859, DOI: 10.1186/1475-2859-5-25
- COTTAZ ET AL: "Genetic engineering of Escherichia coli for the production of N<I>,N<II>-diacetylchitobiose (chitinbiose) and its utilization as a primer for the synthesis of complex carbohydrates", METABOLIC ENGINEERING, ACADEMIC PRESS, US, vol. 7, no. 4, 1 July 2005 (2005-07-01), pages 311 - 317, XP005052642, ISSN: 1096-7176, DOI: 10.1016/J.YMBEN.2005.05.004
- BETTLER E ET AL: "The living factory: in vivo production of N-acetyllactosamine containing carbohydrates in E. coli", GLYCOCONJUGATE JOURNAL, CHAPMAN & HALL, vol. 16, no. 3, 1 March 1999 (1999-03-01), pages 205 - 212, XP002134857, ISSN: 0282-0080, DOI: 10.1023/A:1007024320183
- NAM SOO HAN ET AL: "Biotechnological production of human milk oligosaccharides", BIOTECHNOLOGY ADVANCES, vol. 30, no. 6, 1 November 2012 (2012-11-01), pages 1268 - 1278, XP055074947, ISSN: 0734-9750, DOI: 10.1016/j.biotechadv.2011.11.003
- MAO ZICHAO ET AL: "Engineering the E-coli UDP-glucose synthesis pathway for oligosaccharide synthesis", BIOTECHNOLOGY PROGRESS, vol. 22, no. 2, March 2006 (2006-03-01), pages 369 - 374, ISSN: 8756-7938
- STEEN JENNIFER A ET AL: "The trehalose phosphotransferase system (PTS) in E. coli W can transport low levels of sucrose that are sufficient to facilitate induction of the csc sucrose catabolism operon.", PLOS ONE 2014, vol. 9, no. 2, 2014, pages e88688, XP093041440, ISSN: 1932-6203, DOI: 10.1371/journal.pone.0088688

## Description

The present invention relates to a bacterial host cell being capable to produce oligosaccharides comprising a terminal galactose-(1->4)-glucose disaccharide, as well as to its use and a method for producing oligosaccharides comprising a terminal galactose-(1->4)-glucose disaccharide.

Human milk represents a complex mixture of carbohydrates, fats, proteins, vitamins, minerals and trace elements. The by far most predominant fraction is represented by carbohydrates, which can be further divided into lactose and more complex oligosaccharides (Human milk oligosaccharides, HMO). Whereas lactose is used as an energy source, the complex oligosaccharides are not metabolized by the infant. The fraction of complex oligosaccharides accounts for up to 1/10 of the total carbohydrate fraction and consists of probably more than 150 different oligosaccharides. The occurrence and concentration of these complex oligosaccharides are specific to humans and thus cannot be found in large quantities in the milk of other mammals, like for example domesticated dairy animals.

The most prominent oligosaccharides are 2'-fucosyllactose and 3'-fucosyllactose which together can contribute up to 1/3 of the total HMO fraction. Further prominent HMOs present in human milk are lacto-*N*-tetraose, lacto-*N*-neotetraose and the lacto-*N*-fucopentaoses I. Besides these neutral oligosaccharides, also acidic HMOs can be found in human milk, like for e.g. 3'-sialyllactose, 6'-sialyllactose and 3-fucosyl-3'-sialyllactose, disialyl-lacto-*N*-tetraose etc. These structures are closely related to epitopes of epithelial cell surface glycoconjugates, the Lewis histoblood group antigens such as Lewis x (LeX), and the structural homology of HMO to epithelial epitopes accounts for protective properties against bacterial pathogens.

The existence of these complex oligosaccharides in human milk is known already for a long time and the physiological functions of these oligosaccharides were subject to medicinal research for many decades. For some of the more abundant human milk oligosaccharides, specific functions have already been identified.

Besides the mentioned local effects in the intestinal tract, HMOs have also been shown to elicit systemic effects in infants by entering the systemic circulation. Also, the impact of HMOs on protein-carbohydrate interactions, e.g., selectin-leukocyte binding, can modulate immune responses and reduce inflammatory responses.

Due to the well-studied beneficial properties of prebiotic oligosaccharides, in particular of HMOs, connected with their limited availability, an effective commercial, i.e. large scale production is highly desirable.

Due to the limited supply and difficulties of obtaining pure fractions of individual human milk oligosaccharides, chemical routes to some of these complex molecules were developed. However, production of human milk oligosaccharides by chemical synthesis, enzymatic synthesis or fermentation proved to be challenging. At least large-scale quantities as well as qualities sufficient for food applications cannot be provided until today through chemical synthesis. In this regard, particularly chemical synthetic routes to human milk oligosaccharides involve several noxious chemicals, which impose the risk to contaminate the final product.

Due to the challenges involved in the chemical synthesis of human milk oligosaccharides, several enzymatic methods and fermentative approaches were developed. Today, for several HMOs such as 2'-fucosyllactose, 3-fucosyllactose, lacto-*N-*tetraose, lacto-*N*-neotetraose, 3'-sialyllactose and 6'-sialyllactose fermentative approaches have been developed, using mainly genetically engineered bacterial strains such as recombinant *Escherichia coli.*

Today, biocatalytic as well as fermentative approaches to HMOs were all based exclusively on lactose as a starting substrate, to which additional monosaccharides are added, as disclosed in, e.g., US 7,521,212 B1 or Albermann et al., (2001) Carbohydr. Res. 334(2) p 97-103. The addition of monosaccharides to lactose can be either catalyzed by glycosyltransferases or glycosidases using suitable activated monosaccharide substrates. In addition, additional monosaccharides can be added to lactose by transgluco-sidase reactions.

In particular the fermentative synthesis of HMOs proved particular efficient, because the required and difficult to synthesized nucleotide activated monosaccharides are provided by the metabolism of the used microbe. However, the use of whole cells for the synthesis of HMOs also bears - in comparison to the biocatalytic approach - several major disadvantages, which are related to transport processes across the cell membrane, metabolic side reactions and the fact that the synthesized oligosaccharides have to be purified from a complex mixture containing, *inter alia,* various polyols (e.g. carbohydrates), nucleic acids, polypeptides, inorganic material etc.

US 2009/082307 A1 discloses the production of oligopolysaccharides by a microbiological process, wherein an exogenous precursor, such as lactose, is added to the medium a host cell is cultivated in, and wherein the precursor is internalized by said cell.

A technical problem related to the use of lactose in fermentative processes is presented by the rearrangement of lactose (beta-D-galactopyranosyl-(1->4)-D-glucose) into lactulose (beta-D-galactopyranosyl-(1->4)-D-fructofuranose) by thermal treatment of lactose. This rearrangement can occur extensively by autoclaving lactose, leading to the rearrangement of several percent of the lactose present in a fermentation medium or lactose fermentation feed to lactulose. However, lactulose represents a non-digestible sugar for humans and is widely used in the treatment of chronic constipation.

Also, the conversion of lactose to lactulose not only leads to the generation of unwanted side products, but furthermore it also generates a potential substrate for glycosylation and the generation of more complex oligosaccharides (e.g. 2'-fucosyl-lactulose). Thus, the generation of lactulose from lactose is leading to the contamination of the desired product with closely related oligosaccharides, which are difficult or even impossible to separate away from the desired product.

Above all, the addition of lactose in particular leads to a well-documented effect known as lactose induced cell killing. This effect is most likely caused by the excessive uptake of lactose and the associated collapse of the proton gradient across the bacterial membrane. In particular the overexpression of the lactose permease gene (e.g. *lacY* of *E. coli*) in combination with the exposure of the recombinant cell can cause considerable growth delay of the recombinant strain and the increased synthesis of cellular polysaccharides (Grube et al., "Hydrogen-producing Escherichia coli strains overexpressing lactose permease: FT-IR analysis of the lactose-induced stress" (2013) Biotechnol. Appl. Biochem. 5, 31.).

Furthermore, today, lactose on commercial scale is entirely derived from whey, a waste product of the dairy industry. Whey is produced in enormous quantities in cheese and casein manufacturing. Thus being derived from the dairy industry there is still concerns related to possible contamination of lactose with prion proteins the causative agent of bovine spongiform encephalopathy (BSE), also widely known as mad cow disease. BSE a fatal neurodegenerative disease in cattle, causing spongy degeneration of the brain and spinal cord, can be transmitted to humans and is there known as variant of Creutzfeldt-Jakob disease. Furthermore lactose, if supplied to a beta-galactosidase positive *E. coli* strain, can be converted to allolactose (beta-D-galactopyranosyl-(1->6)-D-glucopyranose), another unwanted contaminant (Huber et al., "Efflux of beta-galactosidase products from Escherichia coli" (1980) J. Bacteriol. 141, 528-533).

In addition lactose is still one of the most expensive components of the fermentation medium and its substitution by glucose, glycerol, sucrose etc. would lead to a more cost effective production of HMOs.

In view of the above, it would be desirable to develop improved means and methods for the production of HMO, which allow overcoming the above mentioned drawbacks of the state of the art.

According to the invention, this and other objects are solved by a bacterial host cell, cell being capable to produce neutral or fucosylated oligosaccharides comprising a terminal galactose-(1->4)-glucose disaccharide, and comprising
(i) at least one recombinant nucleic acid sequence encoding for a protein having a beta-1,4-galactosyltransferase activity and being able to galactosylate a free glucose monosaccharide to intracellularly generate lactose,
(ii) at least one recombinant nucleic acid sequence encoding a glycosyltransferase that is selected from at least one of the following: a fucosyltransferase, a sialyltransferase, a glucosamyltransferase, a galactosyltransferase,
(iii) a genetic modification that leads to an overexpression of the UDP-galactose-4'epimerase, or the UDP-galactose-4'epimerase in combination with the glucose-1-phosphate-1-uridylyltransferase to intracellularly generate UDP-galactose,
(iv) at least one recombinant nucleic acid sequence encoding a protein having an invertase activity and being able to convert sucrose to intracellularly generate fructose and glucose,
and which bacterial host cell is capable of generating the oligosaccharide without the exogenous addition of lactose, glucose and fructose.

The object is further solved by a method for producing at least one oligosaccharide of interest by total fermentation through genetically modified host cells, the oligosaccharide of interest comprising a terminal galactose-(1->4)-glucose disaccharide, and the method comprising the steps of:
a) obtaining a bacterial host according to the invention,
b) culturing said bacterial host cell, wherein said culturing is performed (i) from a carbon source that is selected from sucrose, wherein the host cell is capable of generating free glucose within the cell from said carbon source, and (ii) without externally supplying lactose during execution of the method, thus producing said oligosaccharide of interest.

The object underlying the invention is completely solved in this way.

With the bacterial cell and the method according to the invention, it is possible to totally ferment a desired oligosaccharide, in particular one that carries a galactose-(1->4)-glucose disaccharide at its reducing end, such as e.g. 2'-fucosyllactose and other HMOs from a simple carbon source such as glucose, sucrose, glycerol, and other c-sources, without the need to externally supply lactose as substrate to the fermentation method of such oligosaccharides.

The simple carbon sources can be used to attain a pool of free glucose within the producing cell, such, that the cell can synthesize lactose. Then, the obtained lactose can either be exported from the synthesizing cell and used by other cells in the fermentation for the synthesis of more complex oligosaccharides, or more advantageously by the synthesizing cell itself for the synthesis of more complex oligosaccharides. The synthesized oligosaccharide products are then exported from the producing cell into the fermentation medium by exporters or permeases.

Presently, the term "nucleic acid" refers to a deoxyribonucleotide or ribonucleotide polymer in either single- or double-stranded form, and unless otherwise limited, encompasses known analogues of natural nucleotides that hybridize to nucleic acids in a manner similar to naturally occurring nucleotides. Unless otherwise indicated, a particular nucleic acid sequence includes the complementary sequence thereof.

Presently, the term "operably linked" as used herein, shall mean a functional linkage between a nucleic acid expression control sequence (such as a promoter, signal sequence, or array of transcription factor binding sites) and a second nucleic acid sequence, wherein the expression control sequence affects transcription and/or translation of the nucleic acid corresponding to the second sequence. Accordingly, the term "Promoter" designates DNA sequences which usually "precede" a gene in a DNA polymer and provide a site for initiation of the transcription into mRNA. "Regulator" DNA sequences, also usually "upstream" of (i.e., preceding) a gene in a given DNA polymer, bind proteins that determine the frequency (or rate) of transcriptional initiation. Collectively referred to as "promoter/regulator" or "control" DNA sequence, these sequences which precede a selected gene (or series of genes) in a functional DNA polymer cooperate to determine whether the transcription (and eventual expression) of a gene will occur. DNA sequences which "follow" a gene in a DNA polymer and provide a signal for termination of the transcription into mRNA are referred to as transcription "terminator" sequences.

The term "recombinant", as used herein with reference to a bacterial host cell indicates that the bacterial cell replicates a heterologous nucleic acid, or expresses a peptide or protein encoded by a heterologous nucleic acid (i.e., a sequence "foreign to said cell"). Recombinant cells can contain genes that are not found within the native (non-recombinant) form of the cell. Recombinant cells can also contain genes found in the native form of the cell wherein the genes are modified and re-introduced into the cell by artificial means. The term also encompasses cells that contain a nucleic acid endogenous to the cell that has been modified without removing the nucleic acid from the cell; such modifications include those obtained by gene replacement, site-specific mutation, and related techniques. Accordingly, a "recombinant polypeptide" is one which has been produced by a recombinant cell. A "heterologous sequence" or a "heterologous nucleic acid", as used herein, is one that originates from a source foreign to the particular host cell (e.g. from a different species), or, if from the same source, is modified from its original form. Thus, a heterologous nucleic acid operably linked to a promoter is from a source different from that from which the promoter was derived, or, if from the same source, is modified from its original form. The heterologous sequence may be stably introduced, e.g. by transfection, transformation, conjugation or transduction, into the genome of the host microorganism cell, wherein techniques may be applied which will depend on the host cell the sequence is to be introduced. Various techniques are known to a person skilled in the art and are, e.g., disclosed in Sambrook et al., Molecular Cloning: A Laboratory Manual, 2nd Ed., Cold Spring Harbor Laboratory Press, Cold Spring Harbor, N.Y. (1989).

Accordingly, a "bacterial host cell" is presently understood as a bacterial cell which has been transformed or transfected, or is capable of transformation or transfection by an exogenous polynucleotide sequence.

Thus, the nucleic acid sequences as used in the present invention, may, e.g., be comprised in a vector which is to be stably transformed/transfected or otherwise introduced into host microorganism cells.

A great variety of expression systems can be used to produce the polypeptides used in the invention. Such vectors include, among others, chromosomal, episomal and virus-derived vectors, e.g., vectors derived from bacterial plasmids, from bacteriophage, from transposons, from yeast episomes, from insertion elements, from yeast chromosomal elements, from viruses, and vectors derived from combinations thereof, such as those derived from plasmid and bacteriophage genetic elements, such as cos-mids and phagemids. The expression system constructs may contain control regions that regulate as well as engender expression. Generally, any system or vector suitable to maintain, propagate or express polynucleotides and to synthesize a polypeptide in a host may be used for expression in this regard. The appropriate DNA sequence may be inserted into the expression system by any of a variety of well-known and routine techniques, such as, for example, those set forth in Sambrook *et al., supra.*

The art is rich in patent and literature publications relating to "recombinant DNA" methodologies for the isolation, synthesis, purification and amplification of genetic materials for use in the transformation of selected host organisms. Thus, it is common knowledge to transform host organisms with "hybrid" viral or circular plasmid DNA which includes selected exogenous (i.e. foreign or "heterologous") DNA sequences. The procedures known in the art first involve generation of a transformation vector by enzymatically cleaving circular viral or plasmid DNA to form linear DNA strands. Selected foreign DNA strands usually including sequences coding for desired protein product are prepared in linear form through use of the same/similar enzymes. The linear viral or plasmid DNA is incubated with the foreign DNA in the presence of ligating enzymes capable of effecting a restoration process and "hybrid" vectors are formed which include the selected exogenous DNA segment "spliced" into the viral or circular DNA plasmid.

The term "nucleic acid sequence encoding..." generally refers to any polyribonucleotide or polydeoxyribonucleotide, which may be unmodified RNA or DNA or modified RNA or DNA, and generally represents a gene which encodes a certain polypeptide or protein. The term includes, without limitation, single- and double-stranded DNA, DNA that is a mixture of single- and double-stranded regions or single-, double- and triple-stranded regions, single- and double-stranded RNA, and RNA that is mixture of single- and double-stranded regions, hybrid molecules comprising DNA and RNA that may be single-stranded or, more typically, double-stranded, or triple-stranded regions, or a mixture of single- and double-stranded regions. The term also encompasses polynucleotides that include a single continuous region or discontinuous regions encoding the polypeptide (for example, interrupted by integrated phage or an insertion sequence or editing) together with additional regions that also may contain coding and/or non-coding sequences.

As used herein, the term "cultivating" means growing a bacterial cell in a medium and under conditions permissive and suitable for the production of the desired oligosaccharide(s). A couple of suitable bacterial host cells as well as mediums and conditions for their cultivation will be readily available for one skilled in the art upon reading the disclosure of this invention in connection with the skilled person's technical and expert background.

It is to be understood that with the invention as disclosed therein, the production of one or more, i.e. two, three, four, etc., oligosaccharides as defined herein is possible, as long as the respective nucleic acids encoding the relevant proteins/enzymes as disclosed herein are comprised in the cell(s).

According to a preferred embodiment, the method according to the invention comprises the step c) recovering the oligosaccharide from the medium and/or the bacterial cells. It is to be understood that step c) follows step b), i.e. the cultivating step, and recovering may be accomplished whenever the desired oligosaccharide as defined herein, or a desired amount thereof, has been reached by the cultivating step.

As used herein, the term "recovering" means isolating, harvesting, purifying, collecting or otherwise separating from the host microorganism culture the oligosaccharide produced by the host microorganism according to the invention.

Further, as used herein term "enzymatic activities" is meant to comprise any molecule displaying enzymatic activity, in particular a protein, and acting as a catalyst to bring about a specific biochemical reaction while remaining unchanged by the reaction. In particular, proteins with enzymatic activities are meant to be comprised by this term, which are able to convert a substrate into a product.

In enzymatic reactions, the molecules at the beginning of the process, called substrates, are converted into different molecules, called products. Almost all chemical reactions in a biological cell need enzymes in order to occur at rates sufficient for life. Since enzymes are selective for their substrates and speed up only a few reactions from among many possibilities, the set of enzymes made in a cell determines which metabolic pathways occur in that cell.

With the bacterial cell, its use and the methods as described herein, it was surprisingly found that total fermentation of a desired oligosaccharide as defined herein can be achieved. The present invention utilizes a bacterial microorganism basically having two specific enzymatic activities, i.e. a beta-1,4-galactosyltransferase activity and at least one glycosyltransferase activity, in a method, where the bacterial host microorganism - growing on a simple carbon source - effectively produces the desired oligosaccharide without adding lactose.

Within the context of the present invention, the expression "without the exogenous addition of lactose" is meant to designate that lactose is not added to the cultivation medium the bacterial host cell according to the invention is cultivated in. "Exogenous" shall be contrary to "endogenously produced" by said cell, and shall mean that the bacterial host cell is not fed with lactose during any time of the method.

Although unmodified microorganisms having the above described enzymatically features can be employed with the present method, according to one aspect of the invention, the bacterial microorganism is a recombinant bacterial microorganism or cell, wherein the recombinant bacterial cell has been transformed to comprise at least the above mentioned enzymatic activities.

The bacterial host cell according to the invention uses the carbon source, i.e. sucrose, as follows: firstly, the carbon source is converted to fructose and glucose by the invertase, and glucose is internalized by the bacterial cell, i.e. transported over the cytoplasmic membrane into the cytosol. In the cytosol, glucose is galactosylated - i.e. a galactose-residue is transferred from an nucleotide activated galactose present (or generated in the cell) to the glucose - by the beta-1,4-galactosyltransferase activity displaying enzyme to produce lactose. Subsequently, the lactose is used as acceptor for the glycosyltransferase activity transferring, e.g., a fucosyl-residue from a nucleotide-activated fucose to the lactose, thus generating fucosyllactose. This process can either be accomplished in the same bacterial cell comprising the beta-1,4-galactosyltransferase activity and at least one glycosyltransferase activity, or the lactose is exported from the cell carrying a beta-1,4-galactosyltransferase activity and used by another (bacterial) cell, which cell is present or added to the medium, in which case the lactose is taken up/internalized by said other cell, which comprises the glycosyltransferase activity using the lactose as acceptor.

The fucosyllactose - or any other produced oligosaccharide as defined herein - is then transported from the cell into the medium the bacterial cell is cultivated in and can be recovered, e.g. isolated, therefrom, and, thus, provided for further use.

Generally, and throughout the present invention, the term "glycosyltransferase activity" or "glycosyltransferase" designates and encompasses enzymes that are responsible for the biosynthesis of disaccharides, oligosaccharides and polysaccharides, and they catalyse the transfer of monosaccharide moieties from an activated nucleotide monosaccharide/sugar (the "glycosyl donor") to a glycosyl acceptor molecule.

Preferably, the glycosyltransferase is selected from a galactosyltransferase, a sialyltransferase, a N-acetylglucosaminyltransferase and a fucosyltransferase.

According to another preferred embodiment, in the bacterial host cell or the method of the invention, the glycosyltransferase is selected from at least one of the following: alpha-1,2-fucosyltransferase, alpha-1,3-fucosyltransferase, beta-1,3-N-acetylglucosamyltransferase, beta-1,3-galactosyltransferase, alpha-2,3-sialyltransferase, alpha-2,6-sialyltansferase, beta-1,4-galactosyltransferase or beta-1,6-galactosyltransferase.

According to preferred embodiments, for the synthesis of 2'-fucosyllactose a suitable 2-fucosyltransferase is expressed in addition to the beta-1,4-galactosyltransferase activity. For the synthesis of 3-fucosyllactose a suitable 3-fucosyltransferase is expressed in addition to the beta-1,4-galactosyltransferase. For the synthesis of 2',3-difucosyllactose, both, a suitable alpha-1,2-fucosyltransferase and a alpha-1,3-fucosyltransferase is expressed in addition to the beta-1,4-galactosyltransferase. For the synthesis of lacto-*N*-tetraose a suitable beta-1,3-*N-*acetylglucosamyltransferase and a beta-1,3-galactosyltransferase is expressed in the producing bacterial host cell in addition to the beta-1,4-galactosyltransferase. For the synthesis of even more complex pentasaccharides such as lacto-*N*-fucopenatose I, the recombinant cell is prepared by introducing nucleic acids to be capable of functional expression of a suitable beta-1,3- -*N*-acetyl-glucosamyltransferase, beta-1,3-galactosyltransferase and alpha-1,2-fucosyltransferase in addition to the beta-1,4-galactosyltransferase.

Non-limiting examples for glycosyltransferases which can be used according to the invention and which shall be part of the invention are, e.g., bacterial fucosyl-transferases, and preferably an alpha-1,2-fucosyltransferase, and more preferably the alpha-1,2-fucosyltransferase encoded by the *wbgL* gene of *E*. *coli*:O126 (genebank acc. No. ADN43847), or an alpha-1,3-fucosyltransferase, and more preferably an alpha-1,3-fucosyltransferase from *Akkermansia muciniphila , Bacteroides fragilis, Helicobacter pylori, Escherichia coli, Salmonella enterica, mammals, Caenorhabditis elegans and Schistosoma mansoni.* Preferably, a glycosyltransferase is used or variants thereof which is disclosed in EP 2 479 263 A1 or from EP 2 439 264 or in WO 2010/142305, the content of which is herewith explicitly referred to and made subject matter of this invention.

"Variant(s)" as the term is used herein, is a polynucleotide or polypeptide that differs from a reference polynucleotide or polypeptide respectively, but retains the essential (enzymatic) properties of the reference polynucleotide or polypepeitde. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques, by direct synthesis, and by other recombinant methods known to the persons skilled in the art.

Within the scope of the present invention, also nucleic acid/polynucleotide and polypeptide polymorphic variants, alleles, mutants, and interspecies homologs are comprised by those terms, that have an amino acid sequence that has greater than about 60% amino acid sequence identity, 65%, 70%, 75%, 80%, 85%, 90%, preferably 91%, 92%, 93%, 94%, 95%, 96%, 97%, 98% or 99% or greater amino acid sequence identity, preferably over a region of at least about 25, 50, 100, 200,500, 1000, or more amino acids, to a polypeptide encoded by a wildtype glycosyltransferase/beta-1,4-galactosyltransferase activity displaying protein.

Accordingly, a "functional fragment" of any of the genes/proteins disclosed therein, is meant to designate sequence variants of the genes/proteins still retaining the same or somewhat lesser activity of the gene or protein the respective fragment is derived from.

Accordingly, the terms "alpha-1,2-fucosyltransferase" or "fucosyltransferase" or a nucleic acid/polynucleotide encoding an "alpha-1,2-fucosyltranferase" or "fucosyltransferase" refer to a glycosyltransferase that catalyzes the transfer of fucose from a donor substrate, for example, GDP-fucose, to an acceptor molecule in an alpha-1,2-linkage. The terms "alpha-1,3-fucosyltranferase or fucosyltransferase" or a nucleic acid/polynucleotide encoding an "alpha-1,3-fucosyltranferase or fucosyltransferase" refer to a glycosyltransferase that catalyzes the transfer of fucose from a donor substrate, for example, GDP-fucose, to an acceptor molecule in an alpha-1,3-linkage. The acceptor molecule can be, e.g., lactose, lacto-*N*-triose II, lacto-*N*-tetraose, lacto-*N*-neotetraose or any derivative thereof.

According to a preferred embodiment, in the bacterial host cell of the invention and as used in the method according to the invention, it is further comprised a nucleic acid sequence encoding at least one of the following additional proteins: a oligosaccharide exporter protein or a permease exporting the synthesized oligosaccharide from the host cell, an invertase, a fructokinase, proteins involved in the biosynthesis of GDP-fucose, GDP-glucose, GDP-mannose, UDP-*N*-acetylglucosamine, UDP-galactose, UDP-*N*-acetylgalactosamine, acid, and/or CMP-sialic acid.

Preferably, the oligosaccharide exporter protein or the permease is a protein capable of exporting a soluble oligosaccharide product out of the producing cell. An exemplary embodiment, which, according to one aspect of the invention, can be used as nucleic acid expressing an exporter protein, is the *yberc0001_9420* gene of *Yersinia bercovieri* ATCC 43970 or variants or homologs or functional fragments thereof. Accordingly, an "exporter protein" or "oligosaccharide exporter" protein" is any protein being able to export an oligosaccharide from a bacterial cell. In this regard it is also referred to the disclosure and particularly to the examples of WO 2010/142305, which describes and discloses oligosaccharide exporters and their use.

Exemplary embodiments for the above mentioned exporter or permease proteins are, e.g., the proteins encoded by the csc-gene cluster, which comprises four genes for sucrose permease, fructokinase, sucrose hydrolase and a transcriptional repressor (*cscB, cscK, cscA,* and *cscR,* respectively), the glucose permease *of Bacillus subtiilis,* glucose permease of *Streptomyces coelicolor or Streptomyces clavuligerus.* Accordingly, in a preferred embodiment the bacterial host cell further comprises the csc-gene cluster.

Exemplary embodiments for proteins involved in the biosynthesis of nucleoside diphosphate sugars are, e.g., a nucleoside diphosphate sugar pyrophosphorylase, a sugar kinase, and preferably, fucosekinase, GDP-fucose-pyrophosphorylase, GDP-mannose 4,6-dehydratase (GMD), GDP-keto-6-deoxymannose 3,5-epimerase, 4-reductase, glucosekinase, glucose-6-phosphate 1-epimerase, phosphoglucomutase, glucose-1-phosphate guanylyltransferase, CMP-sialate synthase.

According to one aspect of the invention, in the bacterial host cell as used and claimed herein, the nucleic acid is an endogenous or a recombinant nucleic acid.

As defined above, the term "endogenous" herein and generally within the field means that the nucleic acid encoding for an enzyme of interest is originating from the bacterial host cell and has not been introduced into said host cell, whereas a "recombinant" nucleic acid has been introduced into said host cell and does not originates from said host cell.

According to another embodiment, the nucleic acid is homologous or heterologous. Presently, and as generally understood in the relevant field, the expression "homologous" refers to a nucleic acid sequence that encodes for a specific product or products and is derived from the same species, in which said nucleic acid sequence is inserted. Accordingly, the term "heterologous" refers to a nucleic acid sequence encoding for a specific product or products and being derived from a species other than those in which said nucleic acid sequence is inserted.

According to another embodiment, the bacterial host cell of the invention further comprises control sequences enabling the controlled overexpression of endogenous or recombinant nucleic acid sequences. As defined above, the term "control sequence" which herein is synonymously used with the expression "nucleic acid expression control sequence", comprises promoter sequences, signal sequence, or array of transcription factor binding sites, which sequences affect transcription and/or translation of a nucleic acid sequence operably linked to the control sequences.

As outlined already further above, the nucleic acid sequence which is used according to the invention, may, e.g., be comprised in a vector which is to be stably transformed/transfected into bacterial host cells. The definitions and detailed description for recombinant production as outlined above shall apply for this paragraph.

In some embodiments, the nucleic acid sequence is placed under the control of an inducible promoter, which is a promoter that directs expression of a gene where the level of expression is alterable by environmental or developmental factors such as, for example, temperature, pH, anaerobic or aerobic conditions, light, transcription factors and chemicals. Such promoters are referred to herein as "inducible" promoters, which allow one to control the timing of expression of the proteins used in the present invention. For *E. coli* -and other bacterial host cells, inducible promoters are known to those of skill in the art.

According to an aspect of the invention, the beta- 1,4-galactosyltransferase is a beta-1,4-galactosyltransferase derived from *Neisseria meningitidis,* and is preferably encoded by a gene selected from the group consisting of LgtB from *Neisseria meningitidis,* or *lex-1* from *Aggregatibacter aphrophilus,* or a functional fragments thereof. It is to be understood, that a person skilled in the art will, upon reading this invention, be able to identify any other enzyme having beta- 1,4-galactosyltransferase and accepting the monosaccharide glucose as substrate and being eventually derived from any other organism, which beta-1,4-galactosyltransferase will operate in the presently described and claimed way.

With the beta-1,4-galactosyltransferase that has been introduced in the bacterial host cell according to the invention, intracellular free glucose, which serves as an acceptor substrate for the β-1,4-galactosyltransferase reaction, is converted to a lactose disaccharide structure for HMO synthesis.

The pool of free glucose for lactose synthesis can be provided by supplying glucose via the cultivation medium.

Alternatively, free glucose can be provided intracellular by using a glucose containing disaccharide or oligosaccharide as carbon source for the recombinant bacterial host cell. The so supplied disaccharide or oligosaccharide is cleaved into monosaccharides thus providing free glucose and other monosaccharides for intracellular HMO synthesis. Besides providing free glucose or glucose containing saccharides, glucose for the oligosaccharide synthesis can be also provided via the metabolism (gluconeogenesis) of the recombinant cell.

Besides a pool of free glucose acting as an acceptor substrate within the bacterial host cell, also an effective supply of, e.g., UDP-galactose is needed. This supply for UDP-galactose can, e.g., be obtained from the cells own metabolism, and the metabolism can be improved by further genetic modification, such as the overexpression of the UDP-galactose-4'-epimerase, or the UDP-galactose-4'-epimerase in combination with the glucose-1-phosphate-1-uridinyltransferase.

Alternatively, UDP-galactose can be obtained by feeding galactose to the HMO producing bacterial host cell via the fermentation medium. The galactose is then taken up by the cell, phosphorylated to galactose-1-phosphate and then converted to UDP-galactose. Genes encoding these enzymatic activities are well known in the literature (Grossiord et al., "Characterization, Expression, and Mutation of the Lactococcus lactis galPMKTE Genes, Involved in Galactose Utilization via the Leloir Pathway" (2003) J. Bacteriol 185(3) 870-878.

For the synthesis of, e.g. lacto-*N*-tetraose, in addition to UDP-galactose also UDP-N-acetylglucosamine is required. UDP-*N*-acetlyglucosamine can be also obtained from the bacterial host cell's own UDP-*N*-acetylglucosamine metabolism. The provision of UDP-*N*-acetylglucosamine for the synthesis of *N*-aectylglucosamine containing oligosaccharides can be improved by the inactivation of the *N*-acetylglucosamine catabolism within the producing cell.

For the synthesis of fucose containing HMO, such as 2'-fucosyllactose, 3-fucosyllactose and 2',3-fucosyllactose but not limited, GDP-L-fucose is needed. GDP-fucose for the synthesis of 2'-fucosyllactose can be provided, e.g., by the action of a bifunctional L-fucokinase/GDP-pyrophosphorylase enzyme, encoded by the *Bacteroidis fragilis fkp* gene - and by supplying L-fucose to the HMO synthesizing cell. Similarly sialic acid can be supplied to the cell that is then taken up by the cell and activated by a CMP-sialate synthase to CMP-sialic acid. CMP-sialic acid (also known as CMP-neuraminic acid) can then be used for the synthesis of sialic acid containing oligosaccharides (such as 3'-sialyllactose, 6'-sialyllactose, 3-fucosyl-3'-sialyllactose).

Alternatively for the provision of GDP-L-fucose genes of the GDP-L-fucose pathway can be overexpressed in order to increase the supply of GDP-L-fucose for the synthesis of fucosylated HMOs such as 2'-fucosyllactose, 3-fucosyllactose, 2',3-difucosyllactos, lacto-*N*-fucopentaose I and other fucosylated HMOs.

According to a preferred embodiment of the bacterial host cell and/or the method according to the invention, the bacterial host cell according to the invention is a *Escherichia coli* strain, a *Lactobacillus* species, a *Corynebacterium* strain, a *Bacillus* strain, a *Lactobacillus* strain, a *Streptococcus* strain, an *Enterococcus* strain, a *Lactococ*cus strain, or a *Clostridium* strain. Preferably, the host cell is a bacterial host cell selected from a *Corynebacterium glutamicum, Clotridium cellulolyticum, Clostridium ljungdahlii, Clostridium autoethanogenum, Clostridium acetobutylicum, Bacillus subtilis, Bacillus megaterium, Lactobacillus casei, Lactobacillus acidophilus, Lactobacillus helveticus, Lactobacillus delbrueckii, Lactococcus lactis* cell. A person skilled in the art will be aware of further bacterial strains when reading the present disclosure.

According to a preferred embodiment, the bacterial host according to the invention is further modified (i) not to express proteins intracellularly degrading precursors of said oligosaccharide, or (ii) to have blocked the expression of proteins intracellulary degrading precursors of said oligosaccharide (iii) or regulated in such a way that they do not interfere with the synthase of the desired oligosaccharides. In a preferred embodiment, said protein is a beta-galactosidase, and preferably lacZ.

Further, with the expression "precursor" compounds are encompassed which are involved in the biosynthetic pathway of the oligosaccharide according to the invention, in particular endogenous precursors, i.e. precursors which are produced and naturally present in the host cell.

As mentioned above, the invention also relates to a method for producing at least one oligosaccharide of interest by total fermentation through genetically modified host cells, the oligosaccharide of interest comprising a terminal galactose-(1->4)-glucose disaccharide, and the method comprising the steps of:
a) obtaining a bacterial host cell of the invention
b) culturing said bacterial host cells from step a), wherein said culturing is per-formed (I) from a carbon source that is sucrose, wherein the host cell is capable of generating free glucose within the cell from said carbon source, and (II) without externally supplying lactose during execution of the method, thus producing said oligosaccharide of interest.

As already for the bacterial host cell explained above, with the method according to the invention, it is possibly to fully or totally ferment an oligosaccharide of interest, without the need of adding precursors of said oligosaccharide, since with the method presented herein, the oligosaccharide can be microbially produced by host cells that grow on simple carbon sources. According to the method of the invention, a bacterial host cell is provided as outlined above, and is cultured on a simple carbon source, i.e. sucrose which is internalized by the bacterial cell, i.e. transported over the cytoplasmic membrane into the cytosol. In the cytosol, the carbon source is converted to fructose and glucose by the invertase, and glucose is galactosylated - i.e. a galactose-residue is transferred from an nucleotide activated galactose present (or generated in the cell) to the glucose - which step is catalyzed by the beta-1,4-galactosyltransferase activity to produce lactose. Subsequently, the lactose is used as acceptor for a glycosyltransferase activity transferring, e.g., a fucosyl-residue from a nucleotide-activated fucose to the lactose, thus generating, e.g., fucosyllactose.

According to the method of the invention, this process can either be accomplished in the same bacterial host cell comprising the beta-1,4-galactosyltransferase activity and at least one glycosyltransferase activity, or only the first step, i.e. step (i), the galactosylation is performed in a first bacterial host cell, the thus generated lactose is exported from the first bacterial cell carrying a beta-1,4-galactosyltransferase activity into the medium the first cell is cultured in, and subsequently used by another, a second bacterial host cell, which second bacterial host cell is present or added to the medium, and the lactose is taken up/internalized by said second bacterial host cell, which comprises the glycosyltransferase activity using the lactose as acceptor.

According to a preferred embodiment of the method and the use according to the invention, the oligosaccharide of interest is a human milk oligosaccharide, preferably a human milk oligosaccharide selected from at least one of the one listed in the attached table 1, preferably selected from 2'-fucosyllactose, 3-fucosyllactose, 2'3-difucosyllactose, 3'-sialyllactose, 6'-sialyllactose, 3-fucosyl-3'-sialyllactose, lacto-*N-*tetraose, lacto-*N*-neotetraose, lacto-*N*-fucopentaose I, lacto-*N*-fucopentaose II, lacto-*N-*fucopentaose III, lacto-*N*-fucopentaose V, lacto-N-difucosylhexose I, lacto-*N-*difucosylhexaose II, lacto-N-sialylpentaose LSTa, LSTb, LSTc.

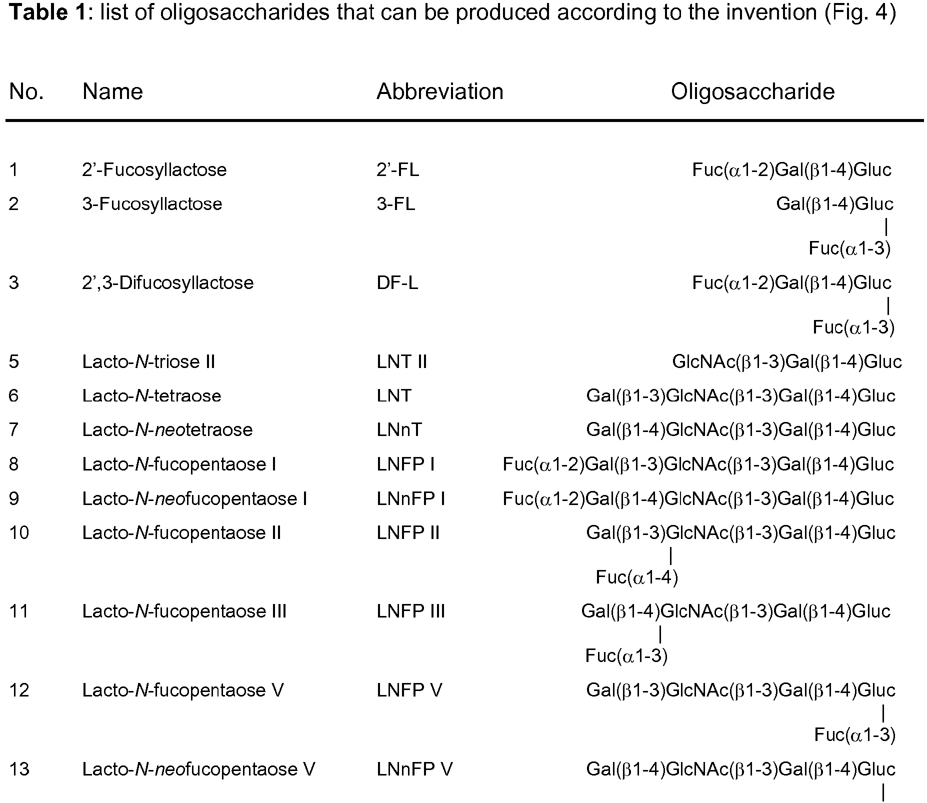

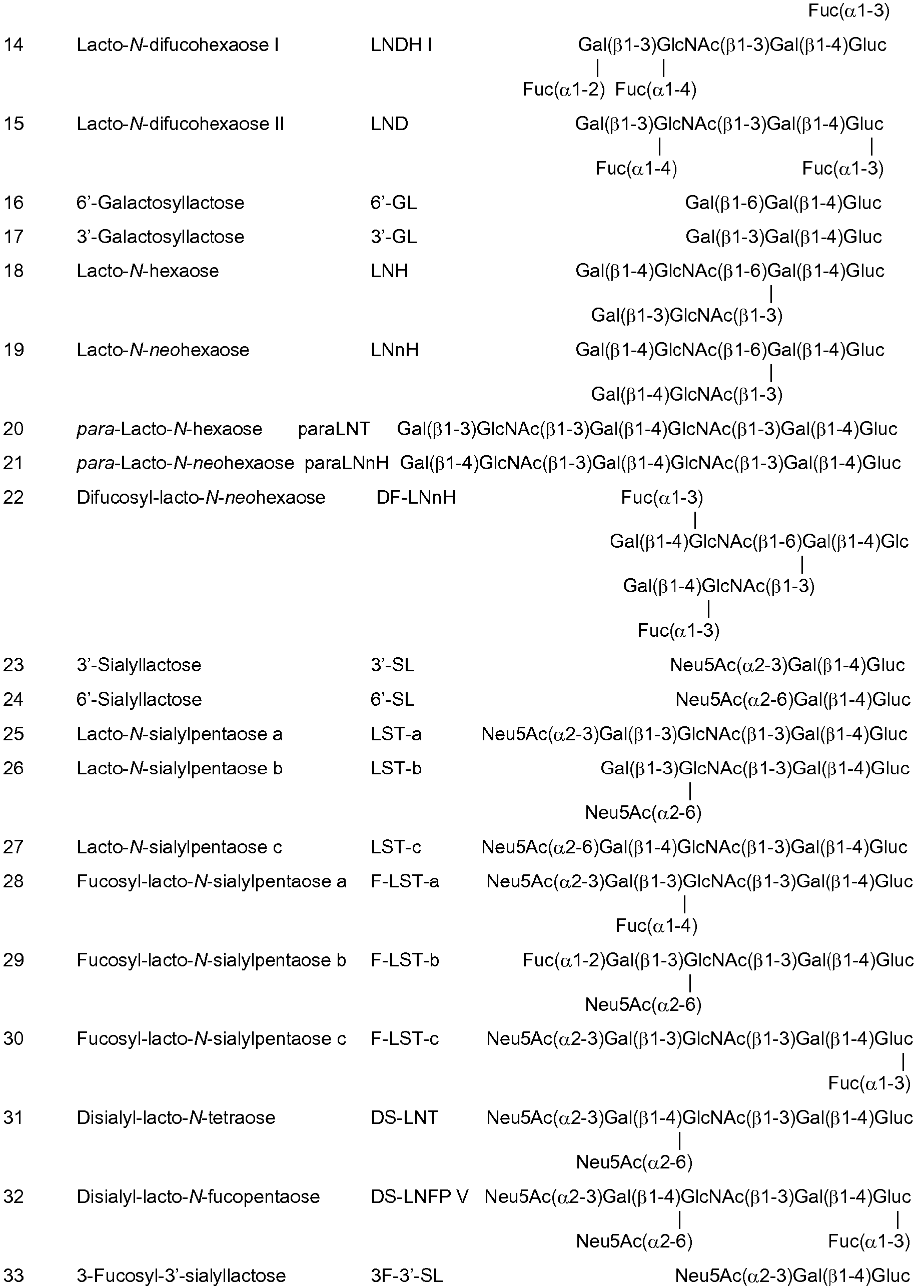

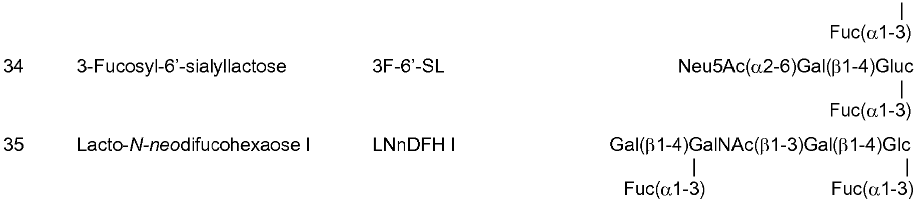

According to a preferred embodiment of the method of the invention in step b), an intracellulary produced nucleotide activated sugar is used by the cell during execution of the method, the nucleotide activated sugar being preferably at least one of the following: GDP-fucose, UDP-*N*-acetylglucosamine, UDP-galactose, UDP-*N-*acetylgalactosamine, CMP-sialic acid
According to another preferred embodiment of the method of the invention, during execution of the method a (non-nucleotide activated) sugar is externally added, wherein the sugar is selected from fucose, glucose, mannose, *N-*acetylglucosamine, galactose, *N*-acetylgalactosamine, sialic acid, and wherein the sugar is nucleotide-activated in said bacterial host cell.

According to one aspect of the invention, the method is a batch or a continuous total fermentation method.

Thus, according to one aspect of the invention, i.e. in a continuous method, a carbon source is constantly added to the medium during the cultivating step of the host cell. By constantly adding the carbon source during the cultivation step, a constant and effective production of the oligosaccharide is accomplished.

According to another aspect, the method according to the invention is or includes a fed-batch fermentation method, with either a constant volume fed-batch culture, where the substrate is fed without diluting the culture, or variable volume fed-batch culture, where the fermentation volume changes with the fermentation time due to the substrate feed.

It is further preferred in an embodiment of the present invention, if the bacterial host cell is further transformed to either lack a gene encoding beta-galactosidase - or comprise a deregulated beta-galactosidase encoding gene-, and L-fucose isomerase, L-fuculose kinase, and UDP-glucose:undecaprenyl phosphate glucose-1-phosphate transferase.

This embodiment has the advantage that intracellular degradation of monosaccharides and production of colonic acid is prevented and in the case of the beta-galctosidase the acceptor molecule is not degraded.

According to a another preferred embodiment, a glycosidase is applied in the method for producing an oligosaccharide, wherein the glycosidase is used for degrading hindering and/or undesired side-products, unused starting substrates and intermediate products generated during the production of the desired oligosaccharide. By means of the glycosidase it can be achieved that, e.g. other (oligo-) saccharides - besides the desired oligosaccharide- , which other (oligo-)saccharides are produced in the microorganism during the synthesis of the desired oligosaccharide, and which other oligosaccharides interfere with the purification step of the desired oligosaccharide, can be metabolised.

One or more glycosidases can be either externally added to the cultivation at the end of the production process according to the invention, which is particularly preferred when endogenous genes of the host microorganism encoding glycosidases have been inactivated or deleted. Alternatively, the glycosidase is endogenously produced by said host microorganism, wherein the nucleic acid sequences encoding the endogenously produced glycosidase is not naturally occurring in said host cell, and wherein said host cell has been stably transformed to express the not naturally occurring glycosidase, and wherein the expression of the glycosidase in the host microorganism is inducable.

According to this embodiment, the oligosaccharide producing microorganism endogenously, i.e. contained in its genome, expresses the glycosidase, e.g. beta-galactosidase, upon external induction, e.g., via temperature or substrate-induced expression, which is otherwise deregulated. This means that during synthesis of the desired oligosaccharide, the glycosidase is deregulated and may be induced, e.g. by temperature or adding a inductor, e.g. tetracyclin, at the end of the fermentation process. The expression of the glycosidase will be induced after a sufficient and/or essentially maximum amount of oligosaccharide has been produced during cultivation of the host microorganism. Subsequently, the expressed glycosidase will degrade undesired saccharide intermediates, substrates, etc., rendering the medium essentially free of the saccharide intermediates or substrates that would otherwise hinder or complicate the purification of the desired oligosaccharide. A couple of suitable inducable expression tools are known in the prior art (see, e.g. Sambrook *et. al,* 1989, *supra*), and one skilled will be able to apply a respectively suitable one for the desired oligosaccharide.

"Regulated" within the present context with reference to a gene is generally understood as a gene, whose expression can be regulated in a controlled fashion, e.g. down- or up-regulated, i.e. the quantity of the synthesised protein encoded by the regulated gene is different, e.g. de-/downregulated or upregulated, from the otherwise unregulated gene.

According to another aspect of the invention, the glycosidase is produced by a second microorganism additionally added to the medium and expressing the glycosidase.

In this embodiment, the glycosidase expressed by the microorganism is either a naturally occurring glycosidase or a glycosidase that is encoded by a nucleic acid sequence that has been stably integrated into the genome of the second microorganism. This embodiment is particularly suitable in a continuous fermentation process for the production of the oligosaccharide, where, e.g., two separate fermentation vessels or containers are provided, whereas one vessel/container is used for the oligosaccharide synthesis reaction and the second vessel/container is essentially employed for the degradation of undesired saccharides.

As mentioned above, the present invention also relates to the use of bacterial host cells of the invention for producing oligosaccharides comprising a terminal galactose-(1->4)-glucose disaccharide by total fermentation through said cells.

Unless defined otherwise, all technical and scientific terms used herein generally have the same meaning as commonly understood by one of ordinary skill in the art to which this invention belongs. Generally, the nomenclature used herein and the laboratory procedures in cell culture, molecular genetics, organic chemistry and nucleic acid chemistry and hybridization described above and below are those well known and commonly employed in the art.

Further advantages follow from the description of the embodiments and the attached drawings.

It goes without saying that the abovementioned features and the features which are still to be explained below can be used not only in the respectively specified combinations, but also in other combinations or on their own, without departing from the scope of the present invention.

Several embodiments of the invention are illustrated in the figures and explained in more detail in the following description. In the figures:

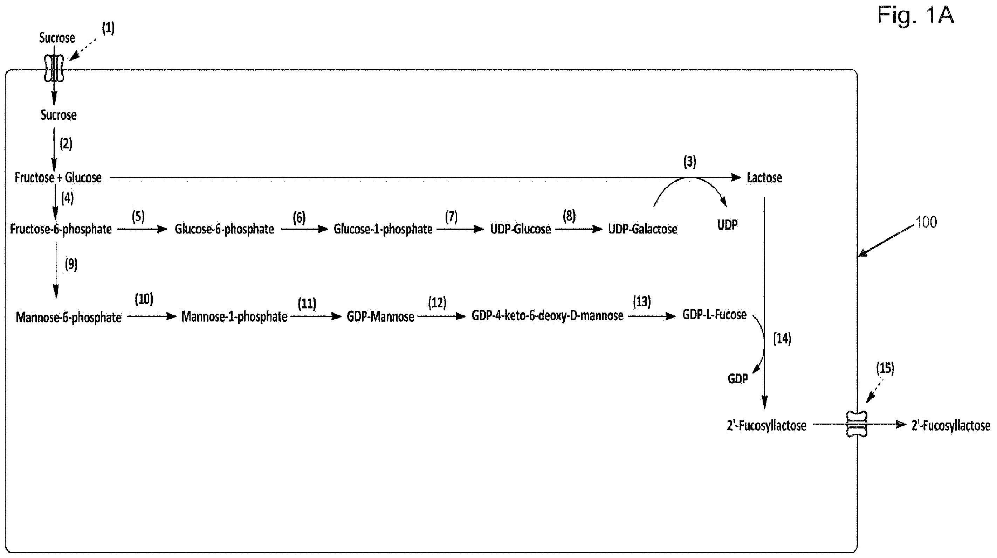

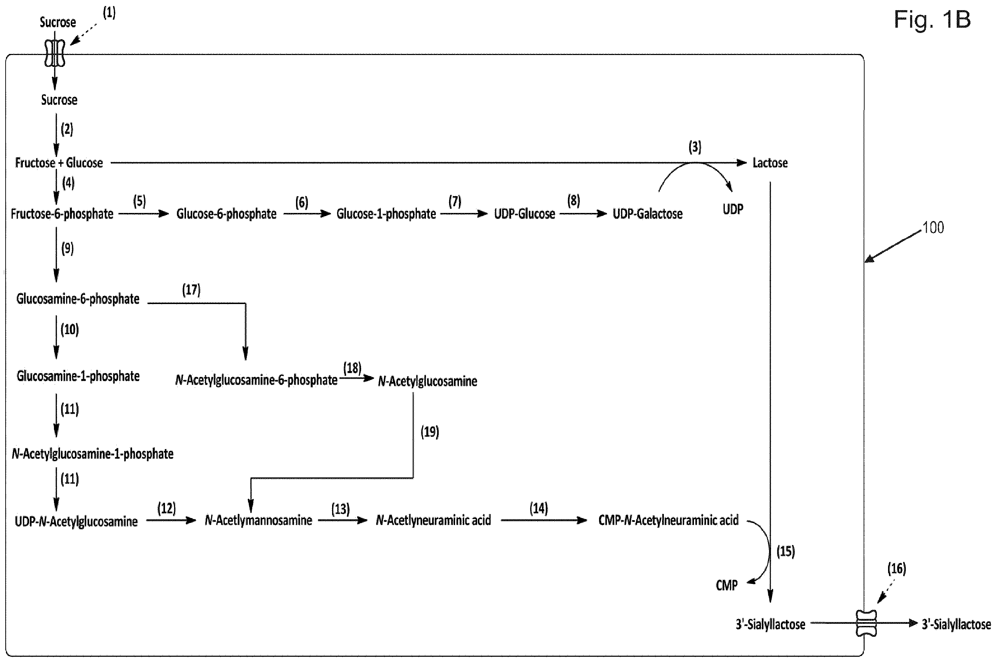

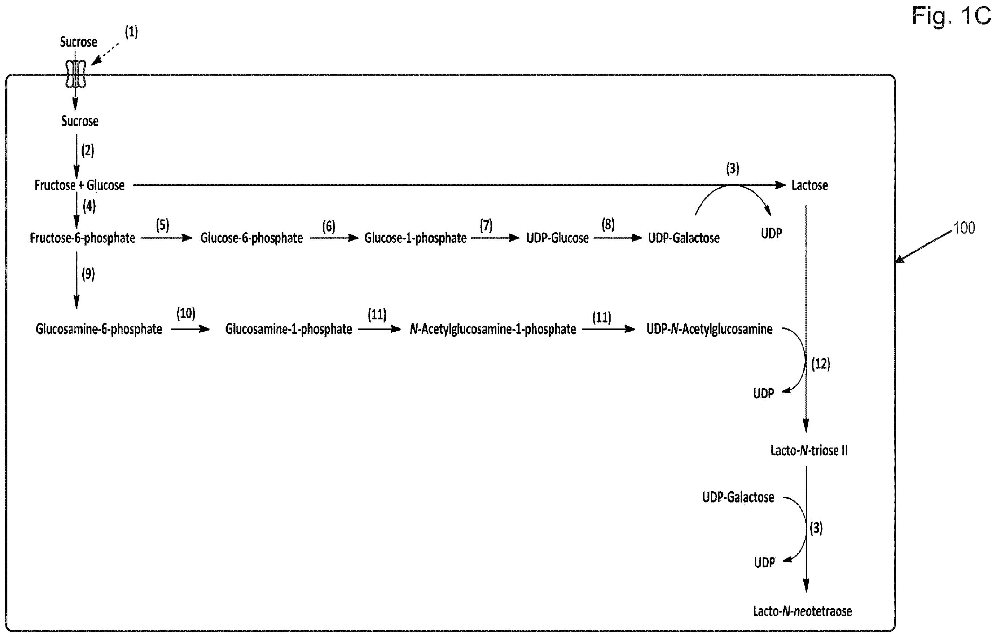

- Fig. 1: shows a schematic drawing for an embodiment of the method according to the invention: production of 2'-fucosyllactose **(A);** 3'-sialyllactose **(B);** and lacto-*N*-neotetraose **(C);**

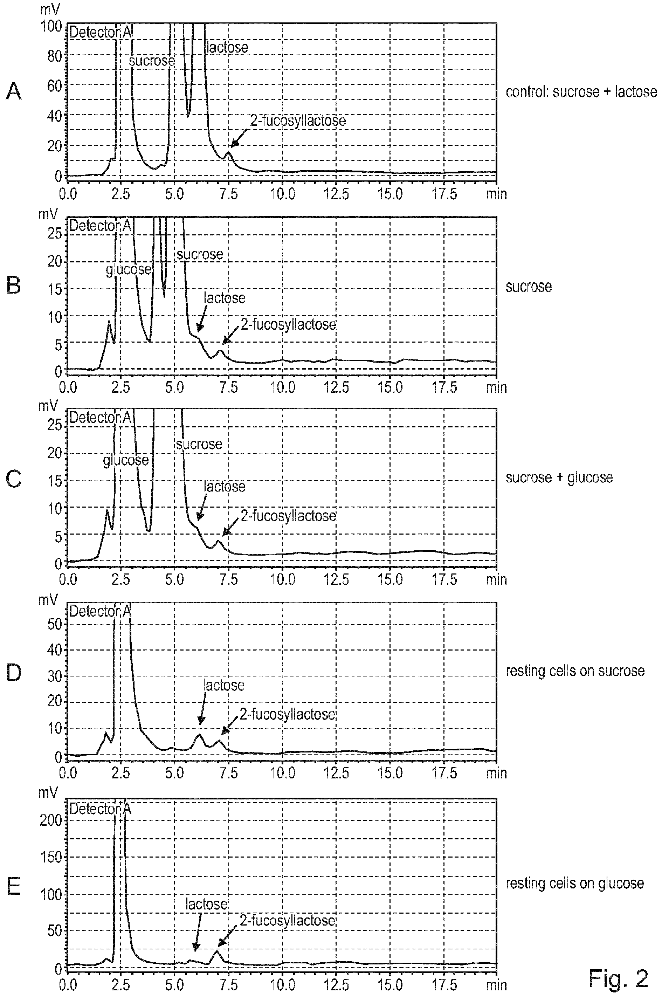

- Fig. 2: shows HPLC analyses of supernatants of cultures of 2'-fucosyllactose producing *E. coli* strains: cells grown in the presents of sucrose and lactose without addition of IPTG **(A);** cells grown on sucrose **(B),** cells grown on sucrose plus glucose **(C);** resting cells: grown in mineral salts media containing sucrose **(D),** and glucose **(E);**

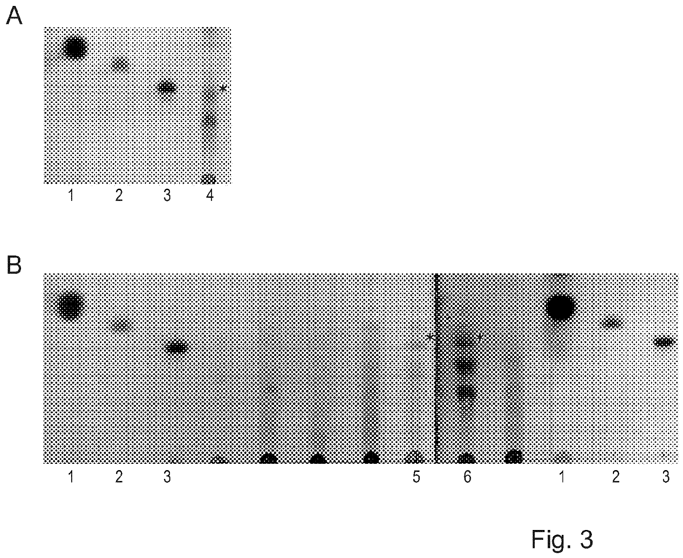

- Fig. 3: shows the results of TLC analyses of culture extracts of lacto-*N*-neotetraose producing *E. coli* BL21(DE3) strain: **(A)** Culture extract of *E. coli* BL21 (DE3) 1046 (4) grown in the presence of glucose; **(B)** Culture extracts of *E. coli* BL21 (DE3) 724 pCDF-*galE* pINT*-malE-lex1* and *E. coli* BL21 (DE3) 724 pCDF-*galE* pINT*-malE-lgtB,* grown on glucose. LNnT;

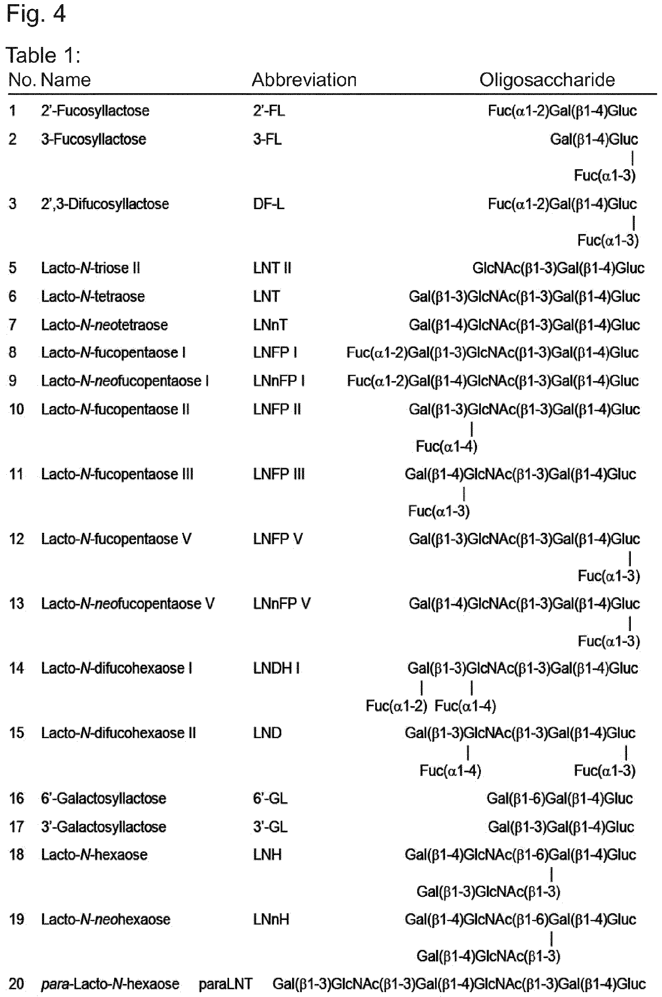

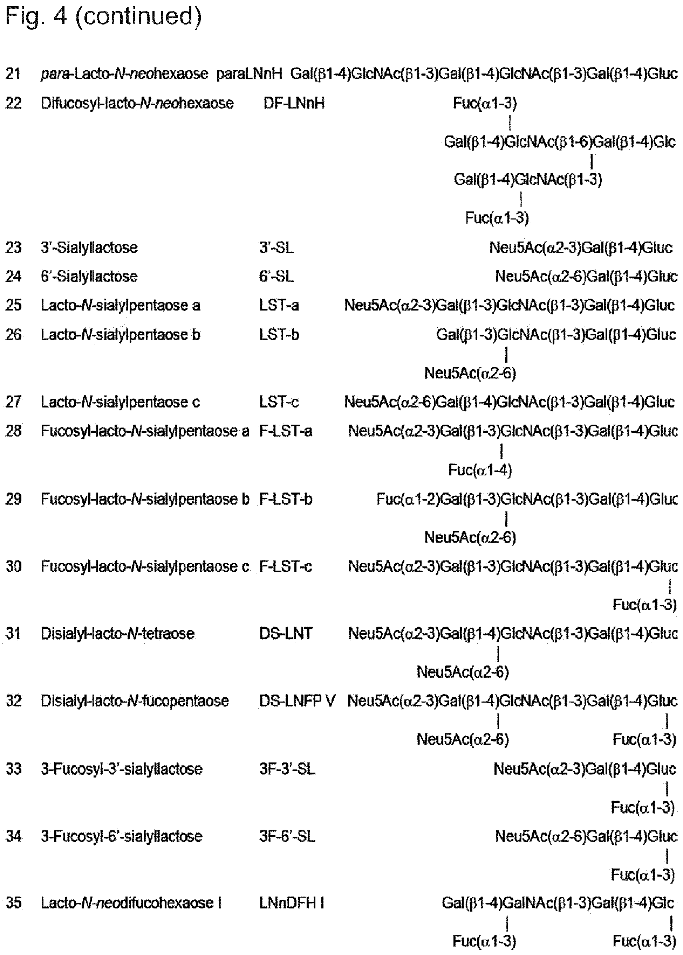

- Fig. 4: shows a list (table 1) of oligosaccharides that can be produced according to the invention; and

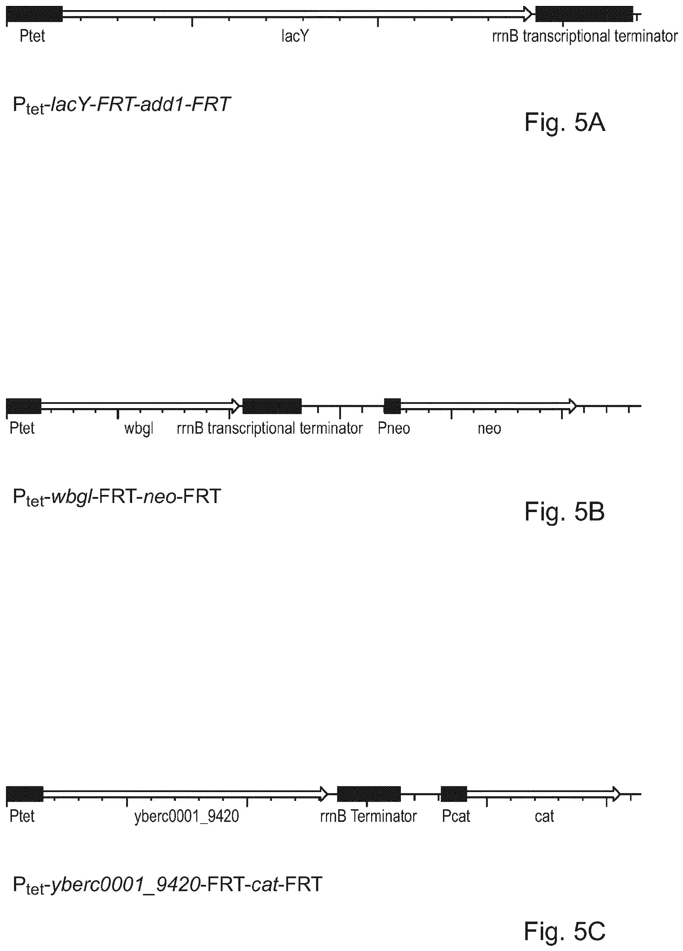

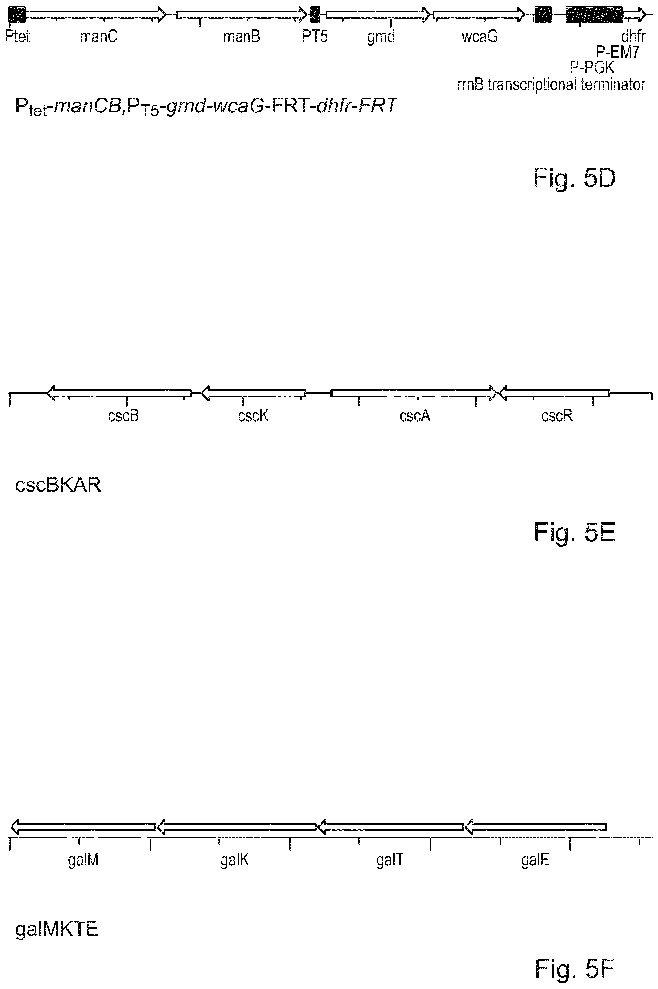

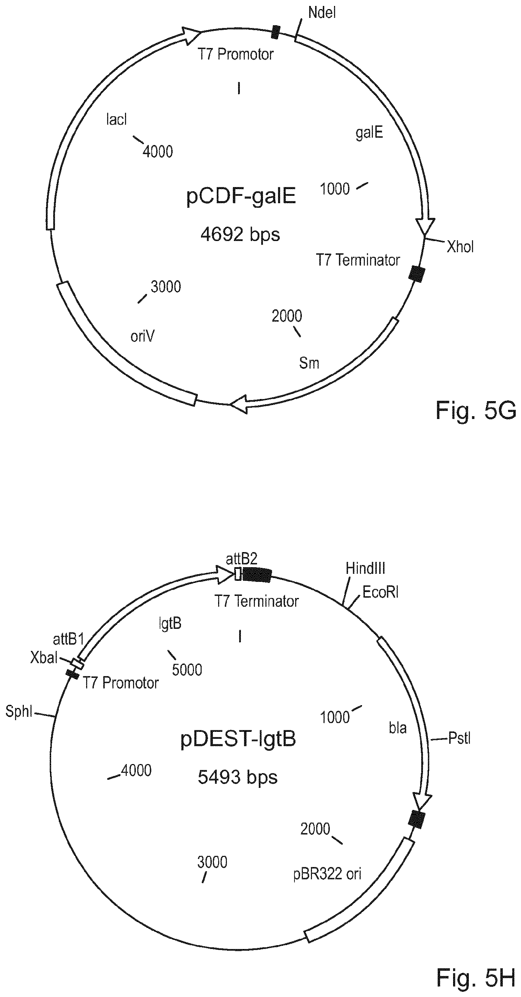

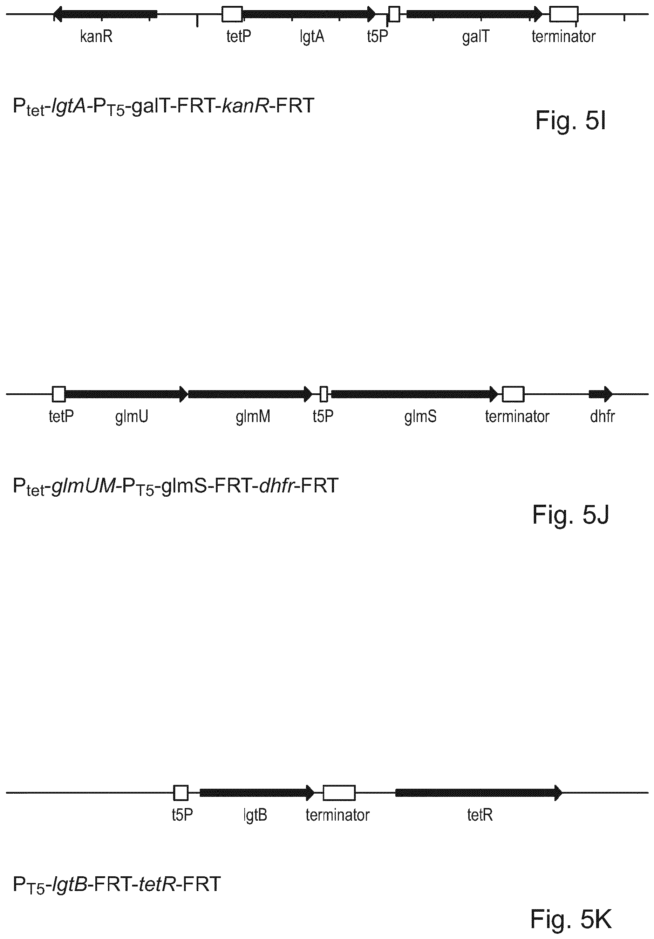

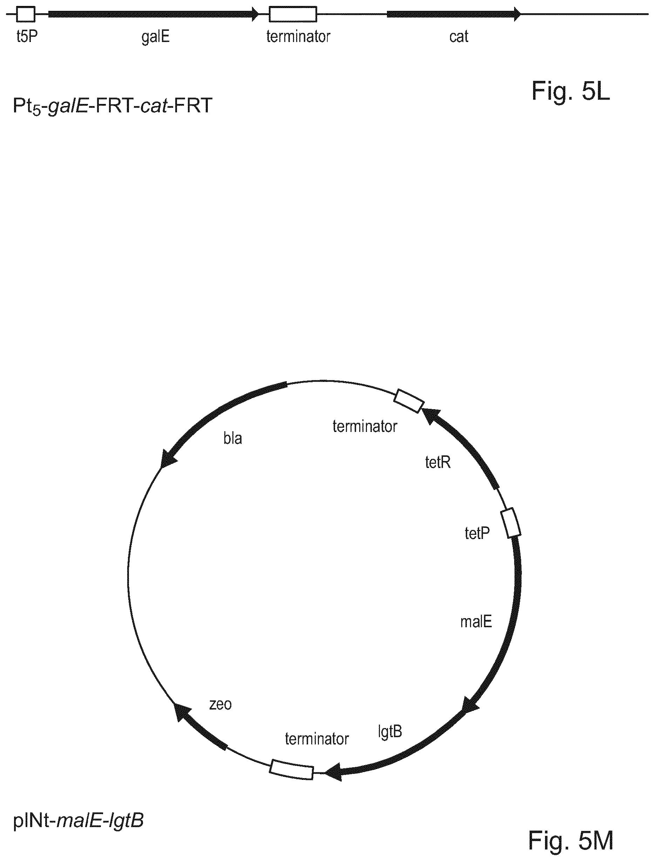

- Fig. 5: shows schematic maps of sequences/plasmids used as exemplary embodiments in the bacterial host cell according to the invention: Pₜₑₜ*-lacY-FRT-add1-FRT* (SEQ ID No. 1 of the attached sequence listing) **(A);** Pₜₑₜ-*wbgL*-FRT-*neo*-FRT SEQ ID No. 2 of the attached sequence listing) (**B**); Pₜₑₜ-*yberc0001_9420*-FRT-*cat-*FRT (SEQ ID No. 3 of the attached sequence listing) **(C);** Pₜₑₜ*-manCB,*P_{T5}*-gmd-wcaG-FRT-dhfr-FRT* (SEQ ID No. 4 of the attached sequence listing) **(D);** cscBKAR (SEQ ID No. 5 of the attached sequence listing) **(E);** galMKTE (SEQ ID No. 6 of the attached sequence listing) **(F);** pCDF-*galE* (SEQ ID No. 7 of the attached sequence listing) **(G);** pDEST-*lgtB* (SEQ ID No. 8 of the attached sequence listing) **(H);** Pₜₑₜ-*lgtA*-P_{T5}-galT-FRT-*kanR-*FRT (SEQ ID No. 9 of the attached sequence listing) **(I);** Pₜₑₜ-*glmUM-*P_{T5}*-glmS*-FRT-*dhfr*-FRT (SEQ ID No. 10 of the attached sequence listing) **(J);** P_{T5}-*lgtB*-FRT-*tetR*-FRT (SEQ ID No. 11 of the attached sequence listing) **(K);** Pₜ₅-*galE*-FRT-*cat*-FRT FRT (SEQ ID No. 12 of the attached sequence listing) **(L);** pINT-*malE-lgtB* (SEQ ID No. 13 of the attached sequence listing) **(M).**

### Detailed Description of preferred embodiments

Fig. 1 shows exemplary pathways for the total fermentation of exemplary oligosaccharides using exemplary embodiments of the bacterial host cells and using the methods of the invention. In figures 1A, 1B and 1C, reference number 100 designates an exemplary bacterial host cell that has been genetically modified according to the invention, with respect to different oligosaccharides of interest.

As can be seen from fig. 1A, which shows the schematic exemplary production, i.e. total fermentation, of 2'-fucosyllactose, sucrose is exemplary used as carbon source. Via a sucrose permease (1), the sucrose is taken up or internalized into the host cell. Within the bacterial host cell, sucrose is degraded by invertase (2) to fructose and glucose. The fructose is phosphorylated by fructokinase (4) to fructose-6-phosphate, which can either be converted by a phosphohexose isomerase (5) to glucose-6-phosphate, fod by a conversion by phosphoglucomutase (6) to glucose-1-phosphate, which in turn is converted by glucose-1-phosphate uridyltransferase (7) to UDP-glucose. UDP-glucose is reacted to UDP-galactose, catalyzed by enzyme UDP-galactose-4-epimerase (8). beta-1,4-galactosyltransferase (3) then catalyzses the reaction UDP-galactose + glucose => UDP + lactose.

Thus, the intracellularly produced lactose can be subsequently converted and used as a basis for oligosacchride production (see examples above). In the embodiment shown in Fig. 1, lactose is converted by enzyme alpha-1,2-fucosyltransferase (14) - using GDP-fucose as donor substrate - to the end product, i.e. 2'-fucosyllactose.

The UDP-fucose may be produced intracellularly (or added): enzyme mannose phosphate isomerase (9) catalyzes the conversion of fructose-6-phosphate to mannose-6-phosphate, which is converted by enzyme phosphomannomutase (10) to mannose-1-phosphate. Enzyme mannose-1-phosphate guanylyltransferase (11) catalyzes the conversion of mannose-1-phosphate to GDP-mannose, which is converted to GDP-4-keto-6-deoxy-D-mannose by GDP-D-mannose 4,6 dehydratase (12). Then, GDP-L-fucose synthase (13) catalyzes the reaction to GDP-fucose, which is used as donor substrate by enzyme alpha-1,2-fucosyltransferas (14) as described above. The thus intracellularly produced 2'-fucosyllactose is subsequently exported by an exporter or permease (15).

Fig. 1B shows the schematic exemplary production, i.e. total fermentation of 3'-sialyllactose using an exemplary embodiment of the bacterial host cell and using the method according to the invention, using the following enzymes: (1) sucrose permease, (2) invertase, (3) β-1,4-galactosyltransferase, (4) fructokinase, (5) glucose-6-phosphate isomerase, (6) phosphoglucomutase, (7) glucose-1-phosphate uridylyltransferase, (8) UDP-galactose-4-epimerase, (9) L-glutamine:D-fructose-6-phosphate aminotransferase, (10) phosphoglucosamine mutase, (11) *N*-acetylglucosamine-1-phosphate uridyltransferase/ glucosamine-1-phosphate acetyl-transferase, (12) UDP-*N*-acetylglucosamin-2-epimerase, (13) sialic acid synthase, (14) CMP-sialic acid synthetase, (15) alpha-2,3-sialyltransferase, (16) sugar efflux transporter, (17) glucosamine-6-phosphateacetyltransferase, (18) phosphatase, (19) *N*-acetylglucosamine-2-epimerase.

Fig. 1C shows the schematic exemplary production, i.e. total fermentation of lacto-N-neotetraose, using an exemplary embodiment of the bacterial host cell and using the method according to the invention, using the following enzymes: (1) sucrose permease, (2) sucrose-6-phosphate hydrolase, (3) beta-1,4-galactosyltransferase, (4) fructokinase, (5) glucosephosphate isomerase, (6) phosphoglucomutase, (7) glucose-1-phosphate uridylyltransferase, (8) UDP-galactose-4-epimerase, (9) L-glutamine:D-fructose-6-phosphate aminotransferase, (10) phosphoglucosamine mutase, (11) *N-*acetylglucosamine-1-phosphate uridyltransferase/ glucosamine-1-phosphate acetyltransferase, (12) beta-1,3-N-acetylglucosamine transferase.

As outlined further above, the bacterial host cell according to the invention may be transformed to comprise - besides the beta-1,4,galactosyltransferade and a glycosyltransferase as defined above, at least one, i.e. one or more, of the enzymes as shown in fig. 1A to 1C, and outlined in the description.

### Example 1

### Development of the E. coli 2'-fucosyllactose production strain

Escherichia coli BL21 (DE3) was used to construct a 2'-fucosyllactose producing strain. Metabolic engineering included mutagenesis and deletions of specific genes, respectively, and genomic integrations of heterologous genes.

The genes *lacZ* and *araA* were inactivated by mutagenesis using mismatch-oligonucleotides as described by Ellis et al., "High efficiency mutagenesis, repair, and engineering of chromosomal DNA using single-stranded oligonucleotides", Proc. Natl. Acad. Sci. USA 98: 6742-6746 (2001).

Genomic deletions were performed according to the method of Datsenko and Warner ("One-step inactivation of chromosomal genes in Escherichia coli K-12 using PCR products", Proc. Natl. Acad. Sci. USA 97:6640-6645 (2000)). To prevent intracellular degradation of L-fucose, genes encoding L-fucose isomerase (*fucI*) and L-fuculose kinase (*fucK*) had been deleted from the genome of the *E. coli* strain BL21 (DE3) strain. Also genes *wzxC-wcaJ* were deleted. *WcaJ* probably encodes a UDP-glucose:undecaprenyl phosphate glucose-1-phosphate transferase catalysing the first step in colanic acid synthesis (Stevenson et al., "Organization of the Escherichia coli K-12 gene cluster responsible for production of the extracellular polysaccharide colonic acid", J. Bacteriol. 178:4885-4893; (1996)); production of colanic acid would compete for GDP-fucose with the fucosyltransferase reaction. In addition the genes *nagA* and *nagB,* coding for *N*-acetylglucosamine-6-phosphate deacetylase and glucosamine-6-phosphate deaminase, respectively, were removed.

Genomic integration of heterologous genes was performed by transposition. Either the EZ-Tn5^{™} transposase (Epicentre, USA) was used to integrate linear DNA-fragments or the hyperactive C9-mutant of the mariner transposase *Himar1* (Lampe et al., "Hyperactive transposase mutants of the Himar1 mariner transposon", Proc. Natl. Acad. Sci. USA 96:11428-11433 (1999)) was used for transposition. To produce EZ-Tn5 transposomes the gene of interest together with a FRT-site flanked antibiotic resistance cassette was amplified with primers 1119 and 1120 (all primers used are listed in table 3); the resulting PCR-product carried on both sites the 19-bp Mosaic End recognition sites for the EZ-Tn5 transposase. For integration using *Himar1* transposase operons of interest together with FRT-site flanked antibiotic resistance cassettes were cloned into vector pEcomar. The transposase is encoded on pEcomar under control of P_{araB}. EZ-Tn5 transposons <Pₜₑₜ-*lacY*-FRT-*aadA*-FRT> (SEQ ID No. 1 of the sequence listing), *<*Pₜₑₜ*-wbgLco-*FRT-*neo*-FRT> (SEQ ID No. 2 of the sequence listing), and < P_{t*e*t}*-yberc0001_9420*-FRT-*cat*-FRT> (SEQ ID No. 3 of the sequence listing) were integrated mediated by EZ-Tn5 transposase.

After successful integration of the gene for the lactose importer LacY from *E. coli* K12 TG1 (acc. no. ABN72583) the resistance gene was eliminated from streptomycin resistant clones by the FLP recombinase encoded on plasmid pCP20 (Datsenko and Warner, "One-step inactivation of chromosomal genes in Escherichia coli K-12 using PCR products", Proc. Natl. Acad. Sci. USA 97:6640-6645 (2000)).

The alpha-1,2-fucosyltransferase gene *wbgL* from *E. coli*:O126 (acc. no. ADN43847) was codon-optimized for expression in *E. coli* and prepared synthetically by GenScript Cooperation (USA). The gene *yberc0001_9420* encoding a sugar efflux transporter of the major facilitator superfamily from *Yersinia bercovieri* ATCC 43970 (acc.no. EEQ08298) was synthesized by GenScript Cooperation (USA) and inserted as EZ-Tn5 *<*Pₜₑₜ*-yberc0001_9420*-FRT-*cat-*FRT> transposon.

To enhance *de novo* synthesis of GDP-fucose, genes encoding phosphomannomutase (*manB*), mannose-1-phosphate guanosyltransferase (*manC*), GDP-mannose-4,6-dehydratase (*gmd*), and GDP-L-fucose synthase (wcaG) from *E. coli* K12 DH5α were overexpressed in the *E. coli* BL21(DE3) strain; the operon *manCB* is under control of Pₜₑₜ, the operon *gmd, wcaG* is transcribed from the P_{T5} promoter. The transposon cassette <Pₜₑₜ-*manCB*-P_{T5}-*gmd*, *wcaG*-FRT-*dhfr*-FRT>>SEQ ID No. 4, flanked by the inverted terminal repeats specifically recognized by the mariner-like element *Himar1* transposase was inserted from pEcomar C9*-manCB-gmd, wcaG-dhfr.*

The csc-gene cluster (SEQ ID No. 5 of the sequence listing) of *E. coli W* (acc. no. CP002185.1) comprises four genes for sucrose permease, fructokinase, sucrose hydrolase, and a transcriptional repressor (genes *cscB, cscK, cscA,* and *cscR,* respectively), that enable the strain to grow on sucrose as sole carbon source. This csc-cluster was integrated into the genome of the *E. coli* BL21(DE3) strain by transposition using plasmid pEcomar-cscABKR.

The gal-operon (*galETKM*) (SEQ ID No. 6 of the sequence listing) was amplified from *E. coli* K12 TG1 using primers 605 and 606 and inserted into the *galM ybhJ* locus of *E. coli* BL21(DE3) strain by homologous recombination facilitated by using the red recombinase helper plasmid pKD46 (Datsenko and Warner, "One-step inactivation of chromosomal genes in Escherichia coli K-12 using PCR products", Proc. Natl. Acad. Sci. USA 97:6640-6645 (2000)).

### Plasmid construction

pCDF-*galE* (SEQ ID No. 7 of the sequence listing): Using genomic DNA of *E. coli* K12 as template, *galE* was amplified using primers 1163 and 1162. The PCR product was purified, restricted with restriction endonucleases *Nde*I and *Xho*I and ligated into the second multiple cloning site of vector pCDF (Novagen), which was cut with the same enzymes.

pDest-*lgtB* (SEQ ID No. 8 of the sequence listing): The gene for the b-1,4-galactosyltransferase *IgtB* from *Neisseria meningitis* was cloned via a gateway reaction (Invitrogen) into vector pDEST14.

**Table 2: Strains and plasmids**

| Strain | Genotype | Ref. |
|---|---|---|
| *E. coli* BL21 (DE3) | F- *omp*T hsdSB(rB-, mB-) *gal dcm* (DE3) | Novagen |
| *E. coli* BL21(DE3) 878 | *E. coli* BL21 (DE3) *ΔlacZ Δara ΔwcaJ ΔfucIK ΔnagAB* harboring genomic integrations of: *galETKM, lacY, cscBKAR, manCB-gmd, wcaG-dhfr, wbglco-neo, yberc0001_9420-cat* | This study |
| pCDF-galE | *galE* integrated into *Ndel Xhol* sites of pCDF | This study |
| pDEST-IgtB | *IgtB* integrated into pDEST expression vector | This study |

**Table 3: oligonucleotides used for PCR**

| Primer | Sequence 5'-3' |
|---|---|
| 605 KI gal fwd | TTACTCAGCAATAAACTGATATTCCGTCAGGCTGG (SEQ ID No. 15) |
| 606 KI gal rev | TTGATAATCTCGCGCTCTTCAGCAGTCAGACTTTCCATATAGAGCGT AATTTCCGTTAACGTCGGTAGTGCTGACCTTGCCGGAGG (SEQ ID No. 16) |
| 1119 ME-for | CTGTCTCTTATACACATCTCCTGAAATTGGCCAGATGATTAATTCCT AATTTTTGTTG (SEQ ID No. 17) |
| 1120 ME rev | CTGTCTCTTATACACATCTCAGCATTACACGTCTTGAGCGATTGTGT AGG (SEQ ID No. 18) |
| 1163 galE_Ndel-for | GATCACATATGAGAGTTCTGGTTACCGGTG (SEQ ID No. 19) |
| 1164 galE_Xhol-rev | GATCACTCGAGTCATTAATCGGGATATCCCTGTGGATGGC (SEQ ID No. 20) |

### 2'-fucosyllactose production by resting cells

*E. coli* BL21 (DE3) 878 harboring plamids pCDF-*galE* and pDEST-*lgtB* was grown in 2YT broth containing ampicillin 100 µg ml⁻¹ and streptomycin 50 µg ml⁻¹ at 30°C. 300 ml fresh 2YT broth containing the same antibiotics were inoculated with 0.3 % (vol/vol) of the overnight grown culture. The cells were grown at 30°C to an OD660nm of 0.3, and gene expression of *galE* and *IgtB* was induced by addition of 0.35 mM IPTG. The cells were sterile harvested by centrifugation after 5 h growth at 30°C, and resuspended in 50 ml sterile mineral salts medium (Samain et al., "Production of O-acetylated and sulphated chitooligosaccharides by recombinant Escherichia coli strains harbouring different combinations of nod genes", J. Biotechnol. 72:33-47 (1999)) containing the above mentioned antibiotics and 2 % (wt/vol) sucrose, and glucose, respectively, as carbon source. The cells were cultivated at 25°C for four days.

2'-fucosyllactose secreted into the supernatant was detected by HPLC.

### Fermentation of 2'-fucosyllactose by overexpression of galE and lgtB

*E. coli* BL21 (DE3) 878 harboring plamids pCDF-*galE* and pDEST-*lgtB* was grown at 30°C in mineral salts medium (Samain et al., "Production of O-acetylated and sulphated chitooligosaccharides by recombinant Escherichia coli strains harbouring different combinations of nod genes", J. Biotechnol. 72:33-47 (1999)) containing ampicillin 100 µg ml⁻¹ and streptomycin 50 µg ml⁻¹ and as carbon source 2% (wt/vol) sucrose, and 1.6 % (wt/vol) sucrose and 0.4 % (wt/vol) glucose, respectively. When the cultures reached an OD660nm of 0.3 gene expression of *galE* and *IgtB* was induced by addition of 0.35 mM IPTG. After induction cells were grown for four days at 25°C.

As a control *E. coli* BL21 (DE3) 878 was grown in mineral salts medium containing 2 % (wt/vol) sucrose as carbon source. When an OD660nm of 0.5 was reached 10 mM lactose was added. Cells were grown for two days at 30°C.

2'-fucosyllactose secreted into the supernatant was detected by HPLC.

### Analysis of culture supernatant and detection of 2'-fucosyllactose by HPLC

Analysis by high performance liquid chromatography (HPLC) was per-formed using a refractive index detector (RID-10A) (Shimadzu, Germany) and a ReproSil Carbohydrate, 5 µm (250mm × 4,6mm) (Dr. Maisch GmbH, Germany) connected to an HPLC system (Shimadzu, Germany). Elution was performed isocratically with acetoni-tril:H₂O (68/32 (v/v)) as eluent at 35°C and a flow rate of 1.4 ml/min. 40 µl of the sample were applied to the column. Samples were filtered (0,22 µm pore size) and cleared by solid phase extraction on an ion exchange matrix (Strata ABW, Phenomenex).

The results of the HPLC analyses are shown in Fig. 2, which shows the HPLC analyses of culture supernatants of 2'-fucosyllactose producing *E. coli* BL21 (DE3) 878 pCDF-*galE* pDEST-*lgtB*. Fig. 2A shows *E. coli* BL21 (DE3) 878 pCDF-*galE* pDEST-*IgtB* grown in the presents of sucrose and lactose without addition of IPTG; Fig. 2B shows cells grown on sucrose, Fig. 2C shows cells grown on sucrose plus glucose; expression of *galE* and *IgtB* was induced with 0.35 mM IPTG at an OD660nm of 0.3.

Fig. 2D and 2E show the HPLC results of resting cells: *E. coli* BL21 (DE3) 878 pCDF-*galE* pDEST-*lgtB* cells were pre-grown in 2YT broth and induced with 0.35 mM IPTG; after harvesting, the cells were grown in mineral salts media containing sucrose (Fig. 2D), and glucose (Fig. 2E), respectively.

As can be seen from the HPLC analyses of Fig. 2, the end product of the exemplary embodiments outlined above, i.e. 2'-fucosyllactose, was efficiently produced upon fermentation of the bacterial host cells according to the invention on sucrose, sucrose plus glucose, resting cells on sucrose, and resting cells on glucose.

### Example 2

### LNnT production by a recombinant E. coli strain by overexpression of galE and a beta-1,4-galactosyltransferase without addition of extracellular lactose

### Development of the E. coli lacto-N-neotetraose production strain

Escherichia coli BL21 (DE3) was used to construct an lacto-*N-*neotetraose (LNnT) producing strain. Metabolic engineering included mutagenesis and deletions of specific genes, respectively, and genomic integrations of heterologous genes.

The genes *lacZ* and *araA* were inactivated by mutagenesis using mismatch-oligonucleotides as described by Ellis et al., "High efficiency mutagenesis, repair, and engineering of chromosomal DNA using single-stranded oligonucleotides", Proc. Natl. Acad. Sci. USA 98: 6742-6746 (2001).

Genomic deletions were performed according to the method of Datsenko and Warner ("One-step inactivation of chromosomal genes in Escherichia coli K-12 using PCR products", Proc. Natl. Acad. Sci. USA 97:6640-6645 (2000)). To prevent intracellular degradation of *N*-acetylglucosamine, genes encoding *N*-acetylglucosamine-6-phosphate deacetylase (*nagA*) and glucosamine-6-phosphate deaminase (*nagB*) were deleted from the genome of the *E. coli* strain BL21 (DE3) strain. Also genes *wzxC-wcaJ* were deleted. *WcaJ* encodes an UDP-glucose:undecaprenyl phosphate glucose-1-phosphate transferase catalysing the first step in colanic acid synthesis (Stevenson et al., "Organization of the Escherichia coli K-12 gene cluster responsible for production of the extracellular polysaccharide colonic acid", J. Bacteriol. 178:4885-4893; (1996)). In addition the genes *fucI* and *fucJ,* coding for L-fucose isomerase and L-fuculose kinase, respectively, were removed.

Genomic integration of heterologous genes was performed by transposition. Either the EZ-Tn5^{™} transposase (Epicentre, USA) was used to integrate linear DNA-fragments or the hyperactive C9-mutant of the mariner transposase *Himar1* (Lampe et al., "Hyperactive transposase mutants of the Himar1 mariner transposon", Proc. Natl. Acad. Sci. USA 96:11428-11433 (1999)) was employed for transposition. To produce EZ-Tn5 transposomes the gene of interest together with a FRT-site flanked antibiotic resistance marker was amplified with primers 1119 and 1120 (all primers used are listed in table 5); the resulting PCR-product carried on both sites the 19-bp Mosaic End recognition sites for the EZ-Tn5 transposase. For integration using *Himar1* transposase expression constructs (operons) of interest were similarly cloned together with a FRT-site flanked antibiotic resistance marker into the pEcomar vector. The pEcomar vector encodes the hyperactive C9-mutant of the mariner transposase *Himar1* under the control of the arabinose inducible promoter P_{araB}.

The expression fragments <Pₜₑₜ-*lacY*-FRT-*aadA*-FRT> and < Pₜ₅-*galE-*FRT-cat-FRT> (SEQ ID No.12 of the sequence listing) were integrated by using the EZ-Tn5 transposase. After successful integration of the gene for the lactose importer *LacY* from *E. coli* K12 TG1 (acc. no. ABN72583) the resistance gene was eliminated from streptomycin resistant clones by the FLP recombinase encoded on plasmid pCP20 (Datsenko and Warner, "One-step inactivation of chromosomal genes in Escherichia coli K-12 using PCR products", Proc. Natl. Acad. Sci. USA 97:6640-6645 (2000)). The N-acetylglucosamine glycosyltransferase gene *IgtA* from *Neisseria meningitidis* MC58 (acc. no. NP_274923) was codon-optimized for expression in *E. coli* and prepared synthetically by gene synthesis. Together with the gene *galT,* encoding a galactose-1-phosphate uridylyltransferase from *E. coli* K-12 substr. MG1655 (acc. no. NP_415279) that was similarly obtained by gene synthesis, *IgtA* was inserted by transposition (SEQ ID No. 9 of the sequence listing) using plasmid pEcomar*-lgtA*-*galT*. To enhance *de novo* synthesis of UDP-*N*-acetylglucosamine, genes encoding L-glutamine:*D*-fuctose-6-phosphate aminotransferase (*glmS*), phosphoglucosamine mutase from *E. coli* K-12 substr. MG1655 (*glmM*) and *N*-acetylglucosamine-1-phosphate uridyltransferase/ glucosamine-1-phosphate acetyltransferase (*glmU*) from *E. coli* K-12 substr. MG1655 (acc. no. NP_418185, NP_417643, NP_418186, respectively) were codon-optimized and obtained by gene synthesis. The operon *glmUM* was cloned under the control of constitutive tetracyclin promoter Pₜₑₜ while *glmS* was cloned under the constitutive P_{T5} promoter. The transposon cassette <Pₜₑₜ-*glmUM*-P_{T5}-glmS-FRT-*dhfr*-FRT> (SEQ ID No. 10 of the sequence listing), flanked by the inverted terminal repeats specifically recognized by the mariner-like element *Himar1* transposase was inserted from pEcomar-glmUM-glmS. The beta-1,4-galactosyltransferase gene *IgtB* from *N. meningitidis* MC58 (acc. no. NP_274922) was codon-optimized for expression in *E. coli* and prepared synthetically. Incorporation into the genome of *E. coli* BL21(DE3) occurred by transposition of the cassette <Pₜ₅-*lgtB*-FRT-*tetR*-FRT> (SEQ ID No. 11 of the sequence listing) from the plasmid pEcomar-lgtB. Furthermore, to improve UDP-galactose synthesis, the UDP-galactose-4-epimerase gene *galE* from *E. coli* K12 DH5α was overexpressed in the *E. coli* BL21(DE3) strain.

The gal-operon (*galETKM*) was amplified from *E. coli* K12 TG1 using primers 605 and 606 and inserted into the *galM ybhJ* locus of *E. coli* BL21(DE3) strain by homologous recombination facilitated by using the red recombinase helper plasmid pKD46 (Datsenko and Warner, "One-step inactivation of chromosomal genes in Escherichia coli K-12 using PCR products", Proc. Natl. Acad. Sci. USA 97:6640-6645 (2000)). Sequences of the heterologous genes and gene clusters are deposit in appendix 1.

### Plasmid construction

Using genomic DNA of *E. coli* K12 DH5α as template, *galE* was amplified using primers 1163 and 1162. The PCR product was purified, restricted with restriction endonucleases *Ndel* and *Xhol* and ligated into the second multiple cloning site of vector pCDF (Novagen), which was cut with the same enzymes. *GalE* is expressed from the IPTG inducible T7 promoter. The genes for the β-1,4-galactosyltransferases *IgtB* from *Neisseria meningitis* (acc. no. NP_274922) and lex1 from *Aggregatibacter aphrophilus NJ8700* (acc. no. YP_003008647) were codon-optimized and synthetically synthesized by GenScript Cooperation (USA). Cloning of both genes occurred by sequence and ligation-independent cloning (Li and Elledge, "Harnessing homologous recombination in vitro to generate recombinant DNA via SLIC.", Nat. Methods. 2007 Mar;4(3):251-6. Epub 2007 Feb 11.) Therefore, the plasmid pINT, harbouring the *malE* gene under control of an anhydro-tetracyline-inducible promoter, was used, enabling the generation of a N-terminal fusion of *malE* with the gene the beta-1,4-galactosyltransferase encoded by the *IgtB* gene or *lex1* gene.

**Table 4: Strains and plasmids**

| Strain | Genotype | Ref. |
|---|---|---|
| *E. coli* BL21 (DE3) | F- *omp*T hsdSB(rB-, mB-) *gal dcm* (DE3) | Novagen |
| *E. coli* BL21(DE3) 1046 | *E. coli* BL21 (DE3) *ΔlacZ Δara ΔwcaJ ΔfucIK ΔnagAB* harbouring genomic integrations of: *galETKM, lacY, lgtA-galT-kanR, glmUM-glmS-dhfr, IgtB-tetR, galE-cat* | This study |
| *E. coli* BL21(DE3) 724 | *E. coli* BL21 (DE3) *ΔlacZ Δara ΔwcaJ ΔfucIK ΔnagAB* harbouring genomic integrations of: *galETKM, lacY, IgtA-galT-kanR, glmUM-glmS-dhfr* | This study |
| pCDF-*galE* | *galE* of *E. coli* K12 integrated into *Ndel Xhol* sites of pCDF | This study |
| pINT*-malE-IgtB* (SEQ ID No. 13) | Gene fusion of *malE* with *IgtB* of *Neisseria meningitis* integrated into vector pINT | This study |
| pINT*-malE-lex1* (SEQ ID No. 14) | Gene fusion of *malE* with *lex-1* of *Aggregatibacter aphrophilus NJ8700* integrated into vector pINT | This study |

**Table 5: Oligonucleotides used for PCR**

| Primer | Sequence 5'-3' |
|---|---|
| 605 KI gal fwd | TTACTCAGCAATAAACTGATATTCCGTCAGGCTGG (SEQ ID No. 21) |
| 606 KI gal rev | TTGTAATCTCGCGCTCTTCACATCAGACTTTCCATATAGAGCGTAA TTTCCGTTAACGTCGGTAGTGCTGACCTTGCCGGAGG (SEQ ID No. 22) |
| 1119 ME-for | CTGTCTCTTATCACATCTCCTGAAATGGCCAGATGTAATTCCTAATT TTTGTTG (SEQ ID No. 23) |
| 1120 ME rev | CTGTCTCTTATCACATCTCACATTACATCTGAGCGATTGTTAGG (SEQ ID No. 24) |
| 1163 galE_Ndel-for | GATCACATATGAGAGTTCTGGTTACCGGTG (SEQ ID No. 25) |
| 1164 galE_Xhol-rev | GATCACTCGAGTCATTAATCGGGATATCCCTGTGGATGGC (SEQ ID No. 26) |
| 5176 lex1 pINT-f | GTCGATGAAGCCCTGAAAGACGCGCAGACTATGCACTTCATTGAA AACAAAAACTTCGTC (SEQ ID No. 27) |
| 5177 lex1 pINT-r | GATGGCCTTTTTGCGTGTCGACGCGGCCGCCTAGATAAACAGGAT GATATTTTTGCCTTG (SEQ ID No. 28) |
| 5178 pINT lex1-f | CAAGGCAAAAATATCATCCTGTTTATCTAGGCGGCCGCGTCGACA CGCAAAAAGGCCATC (SEQ ID No. 29) |
| 5179 pINT lex1-r | GACGAAGTTTTTGTTTTCAATGAAGTGCATAGTCTGCGCGTCTTTC AGGGCTTCATCGAC (SEQ ID No. 30) |
| 5180 IgtB plNT-f | GTCGATGAAGCCCTGAAAGACGCGCAGACTATGGCCCAAAACCAC GTTATCAGCTTAGCT (SEQ ID No. 31) |
| 5181 IgtB plNT-r | GATGGCCTTTTTGCGTGTCGACGCGGCCGCTTATTGGAAAGGCAC AATGAACTGTTCGCG (SEQ ID No. 32) |
| 5182 pINT IgtB-f | CGCGAACAGTTCATTGTGCCTTTCCAATAAGCGGCCGCGTCGACA CGCAAAAAGGCCATC (SEQ ID No. 33) |
| 5183 pINT IgtB-r | AGCTAAGCTGATAACGTGGTTTTGGGCCATAGTCTGCGCGTCTTTC |
| | AGGGCTTCATCGAC (SEQ ID No. 34) |

### Batch fermentation of E. coli BL21 (DE3) 1046

*E. coli* BL21 (DE3) 1046 was grown for 48 hours at 30°C in mineral salts medium (Samain et al., "Production of O-acetylated and sulphated chitooligosaccharides by recombinant Escherichia coli strains harbouring different combinations of nod genes", J. Biotechnol. 72:33-47 (1999)) containing 2 % (wt/vol) glucose as sole carbon and energy source. Cells were harvested and mechanically disrupted. The production of LNnT was detected by thin layer chromatography.

### Batch fermentation of E. coli BL21 (DE3) 724 pCDF-galE pINT-malE-lgtB/pINT-malE-lex1

*E. coli* BL21 (DE3) 724 harbouring plamids pCDF-*galE* and either pINT-malE*-lgtB* or pINT-malE-*lex1* was grown at 30°C in mineral salts medium (Samain et al., "Production of O-acetylated and sulphated chitooligosaccharides by recombinant Escherichia coli strains harbouring different combinations of nod genes", J. Biotechnol. 72:33-47 (1999)) supplemented with 2 % (wt/vol) glucose, ampicillin 100 µg ml⁻¹ and zeocin 40 µg ml⁻¹. When the cultures reached an OD660nm of 0.1, gene expression of the *galE* gene and the β1,4-galactosyltransferase was induced by addition of 0.3 mM IPTG and 200 ng/ml anhydrotetracycline. After incubation for 48 hours at 30°C in shaking flasks cells were harvested and mechanically disrupted. LNnT was detected by thin layer chromatography.

### Detection of lacto-N-neotetraose (LnNT) by thin layer chromatography (TLC)

Cells war mechanically disrupted using glass beads. Subsequently, samples were applied on TLC Silica Gel 60 F₂₅₄ (Merck, Germany). The mobile phase was composed of acetone:butanol:acetic acid:water (35:35:7:23).

### TLC analyses of culture extracts of lacto-N-neotetraose producing E. coli BL21(DE3) strain

Fig. 3 shows TLC (thin layer chromatography) analyses of culture extracts of lacto-*N*-neotetraose producing *E. coli* BL21(DE3) cells. Fig. 2A shows the results for a culture extract of *E. coli* BL21 (DE3) 1046 (4) grown in the presence of glucose, with the following standards: lactose (lane 1), lacto-*N*-triose II (lane 2) and lacto-*N-*neotetraose (lane 3). Fig. 3B shows the results for culture extracts of *E. coli* BL21 (DE3) 724 pCDF-*galE* pINT-*malE-lex1* (lane 5) and *E. coli* BL21 (DE3) 724 pCDF-*galE* pINT-*malE-lgtB* (lane 6) grown on glucose. LN*n*T product formation in the samples is marked with asterisks.

Thus, the results presented in Fig. 3 show the efficient production of lacto-*N*-neotetraose that was effected with the cells and the methods according to the invention.

## Claims

1. A bacterial host cell being capable to produce neutral or fucosylated oligosaccharides comprising a terminal galactose-(1->4)-glucose disaccharide, and comprising
(i) at least one recombinant nucleic acid sequence encoding for a protein having a beta-1,4-galactosyltransferase activity and being able to galactosylate a free glucose monosaccharide to intracellularly generate lactose,
(ii) at least one recombinant nucleic acid sequence encoding a glycosyltransferase that is selected from at least one of the following: a fucosyltransferase, a sialyltransferase, a glucosamyltransferase, a galactosyltransferase,
(iii) a genetic modification that leads to an overexpression of the UDP-galactose- 4'epimerase, or the UDP-galactose-4'-epimerase in combination with the glucose- 1-phosphate-1-uridylyltransferase to intracellularly generate UDP-galactose,
(iv) at least one recombinant nucleic acid sequence encoding a protein having an invertase activity and being able to convert sucrose to intracellularly generate fructose and glucose,
and which bacterial host cell is capable of generating the oligosaccharide without the exogenous addition of lactose, glucose and fructose.

2. The bacterial host cell of claim 1, wherein the glycosyltransferase is selected from at least one of the following: alpha-1,2-fucosyltransferase, alpha-1,3-fucosyltransferase, beta-1,3-N-acetyl-glucosamyltransferase, beta-1,3-galactosyltransferase, beta-1,4-galactosyltransferase, alpha-2.6-sialyltransferase, alpha-2,3-sialyltransferase, beta-1,6-galactosylfransferase, or a functional fragment thereof.

3. The bacterial host cell of claim 1 or 2, further comprising a nucleic acid sequence encoding at least one of the following additional proteins: an exporter protein or a permease exporting the synthesized oligosaccharide from the host cell.

4. The bacterial host cell of claim 3, wherein the exporter protein is the sugar efflux transporter encoded by the *yberc0001_9420* gene of *Yersinia bercovieri* ATCC 43970 or a functional fragment thereof.

5. The bacterial host cell of any of claims 1 to 4, further comprising a nucleic acid sequence encoding at least one of the following additional proteins: a fructokinase, proteins involved in the biosynthesis of GDP-fucose, GDP-mannose, UDP-glucose, UDP-N-acetylglucosamine, UDP-galactose, UDP-N-acetylgalactosamine, UDP-glucuronic acid, CMP-sialic acid.

6. The bacterial host cell of claim 4 or 5, wherein the nucleic acid is an endogenous or a recombinant nucleic acid.

7. The bacterial host cell of any of claims 4 to 6, wherein the nucleic acid is homologous or heterologous.

8. The bacterial host cell of any of the preceding claims further comprising control sequences enabling the controlled overexpression of endogenous or recombinant nucleic acid sequences.

9. The bacterial host cell of any of the preceding claims, wherein the beta-1,4-galactosyltransferase is encoded by a gene selected from the group consisting of *IgtB* from *Neisseria meningitidis,* and *lex-1* from *Aggregatibacter aphrophilus NJ8700,* or functional fragments thereof.

10. The bacterial host cell of any of the preceding claims, wherein the bacterial host cell is a *Escherichia coli* strain, a *Lactobacillus* species, a *Corynebacterium glutamicum* strain, or a Clostridium strain.

11. The bacterial host cell of any of the preceding claims, further comprising at least one of the genes of the *E. coli W csc* gene cluster.

12. The bacterial host cell of any of the preceding claims, wherein the bacterial host cell is modified not to express proteins intracellularly degrading precursors of said oligosaccharide.

13. The bacterial host cell of any of the preceding claims, wherein the bacterial host cell is further genetically modified to contain a nucleic acid sequence encoding a glycosidase for the specific degradation of interfering oligosaccharides, such as intermediates, side products or endogenous oligosaccharides generated by bacterial host cell, wherein the expression of said glycosidase is under control of a regulatory sequence.

14. Method for producing at least one neutral or fucosylated oligosaccharide of interest by total fermentation through genetically modified host cells, the oligosaccharide of interest comprising a terminal galactose-(1->4)-glucose disaccharide, and the method comprising the steps of:
a) obtaining a bacterial host cell as claimed in any of the preceding claims;
b) culturing said bacterial host cells from step a), wherein said culturing is performed (I) from a carbon source that is sucrose, wherein the host cell is capable of generating free glucose within the cell from said carbon source, and (II) without externally supplying lactose during execution of the method, thus producing said oligosaccharide of interest.

15. Use of a bacterial host cell as claimed in any of claims 1 to 11 for producing oligosaccharides comprising a terminal galactose-(1->4)-glucose disaccharide by total fermentation through said cells.

## Patentansprüche

1. Bakterielle Wirtszelle, die in der Lage ist, neutrale oder fucosylierte Oligosaccharide zu produzieren, umfassend ein terminales Galactose-(1->4)-Glucose-Disaccharid, und umfassend
(i) zumindest eine rekombinante Nukleinsäuresequenz, die für ein Protein mit einer beta-1,4-Galactosyltransferase-Aktivität codiert und in der Lage ist, ein freies Glucosemonosaccharid zu galactosylieren, um intrazellulär Lactose zu erzeugen,
(ii) zumindest eine rekombinante Nukleinsäuresequenz, die eine Glycosyltransferase codiert, die ausgewählt ist aus zumindest einem des Folgenden: einer Fucosyltransferase, einer Sialyltransferase, einer Glucosamyltransferase, einer Galactosyltransferase,
(iii) eine genetische Modifikation, die zu einer Überexpression der UDP-Galactose-4'-Epimerase oder der UDP-Galactose-4'-Epimerase in Kombination mit der Glucose-1-Phosphat-1-Uridylyltransferase führt, um intrazellulär UDP-Galactose zu erzeugen,
(iv) zumindest eine rekombinante Nukleinsäuresequenz, die ein Protein codiert, das eine Invertase-Aktivität aufweist und in der Lage ist, Saccharose umzusetzen, um intrazellulär Fructose und Glucose zu erzeugen,
und wobei die bakterielle Wirtszelle in der Lage ist, das Oligosaccharid ohne die exogene Hinzufügung von Lactose, Glucose und Fructose zu erzeugen.

2. Bakterielle Wirtszelle nach Anspruch 1, wobei die Glycosyltransferase ausgewählt ist aus zumindest einem des Folgenden: alpha-1,2-Fucosyltransferase, alpha-1,3-Fucosyltransferase, beta-1,3-N-Acetylglucosamyltransferase, beta-1,3-Galactosyltransferase, beta-1,4-Galactosyltransferase, alpha-2,6-Sialyltransferase, alpha-2,3-Sialyltransferase, beta-1,6-Galactosylfransferase oder einem funktionellen Fragment davon.

3. Bakterielle Wirtszelle nach Anspruch 1 oder 2, ferner umfassend eine Nukleinsäuresequenz, die zumindest eines der folgenden zusätzlichen Proteine codiert: ein Exporterprotein oder eine Permease, die das synthetisierte Oligosaccharid aus der Wirtszelle exportieren.

4. Bakterielle Wirtszelle nach Anspruch 3, wobei das Exporterprotein der Zuckerausflusstransporter ist, der durch das *yberc0001_9420-Gen* von *Yersinia bercovieri* ATCC 43970 oder ein funktionelles Fragment davon codiert wird.

5. Bakterielle Wirtszelle nach einem der Ansprüche 1 bis 4, ferner umfassend eine Nukleinsäuresequenz, die zumindest eines der folgenden zusätzlichen Proteine codiert: eine Fructokinase, Proteine involviert an der Biosynthese von GDP-Fucose, GDP-Mannose, UDP-Glucose, UDP-N-Acetylglucosamin, UDP-Galactose, UDP-N-Acetylgalactosamin, UDP-Glucuronsäure, CMP-Sialinsäure.

6. Bakterielle Wirtszelle nach Anspruch 4 oder 5, wobei die Nukleinsäure eine endogene oder eine rekombinante Nukleinsäure ist.

7. Bakterielle Wirtszelle nach einem der Ansprüche 4 bis 6, wobei die Nukleinsäure homolog oder heterolog ist.

8. Bakterielle Wirtszelle nach einem der vorhergehenden Ansprüche, ferner umfassend Kontrollsequenzen, welche die kontrollierte Überexpression von endogenen oder rekombinanten Nukleinsäuresequenzen ermöglichen.

9. Bakterielle Wirtszelle nach einem der vorhergehenden Ansprüche, wobei die beta-1,4-Galactosyltransferase durch ein Gen ausgewählt aus der Gruppe bestehend aus *IgtB* aus *Neisseria meningitidis,* und *lex-1* aus *Aggregatibacter aphrophilus NJ8700* oder funktionellen Fragmenten davon codiert ist.

10. Bakterielle Wirtszelle nach einem der vorhergehenden Ansprüche, wobei die bakterielle Wirtszelle ein *Escherichia-coli-*Stamm, eine *Lactobacillus*-Spezies, ein *Corynebacteriumglutamicum*-Stamm oder ein Clostridium-Stamm ist.

11. Bakterielle Wirtszelle nach einem der vorhergehenden Ansprüche, ferner umfassend zumindest eines der Gene des Genclusters *E. coli W csc.*

12. Bakterielle Wirtszelle nach einem der vorhergehenden Ansprüche, wobei die bakterielle Wirtszelle modifiziert ist, um Proteine, die Vorläufer des Oligosaccharids intrazellulär abbauen, nicht zu exprimieren.

13. Bakterielle Wirtszelle nach einem der vorhergehenden Ansprüche, wobei die bakterielle Wirtszelle ferner genetisch modifiziert ist, um eine Nukleinsäuresequenz zu enthalten, die eine Glycosidase für den spezifischen Abbau von interferierenden Oligosacchariden, wie Zwischenprodukten, Nebenprodukten oder endogenen Oligosacchariden erzeugt durch bakterielle Wirtszelle, codiert, wobei die Expression der Glycosidase unter Kontrolle einer regulatorischen Sequenz ist.

14. Verfahren zum Produzieren von zumindest einem neutralen oder fucosylierten Oligosaccharid von Interesse durch vollständige Fermentation durch genetisch modifizierte Wirtszellen, wobei das Oligosaccharid von Interesse ein terminales Galactose-(1->4)-Glucose-Disaccharid umfasst, und das Verfahren die folgenden Schritte umfasst:
a) Erhalten einer bakteriellen Wirtszelle nach einem der vorhergehenden Ansprüche;
b) Kultivieren der bakteriellen Wirtszellen aus Schritt a), wobei das Kultivieren (I) aus einer Kohlenstoffquelle, die Saccharose ist, wobei die Wirtszelle in der Lage ist, freie Glucose innerhalb der Zelle aus der Kohlenstoffquelle zu erzeugen, und (II) ohne externes Zuführen von Lactose während Ausführung des Verfahrens, wodurch das Oligosaccharid von Interesse produziert wird, durchgeführt wird.

15. Verwendung einer bakteriellen Wirtszelle nach einem der Ansprüche 1 bis 11 zum Produzieren von Oligosacchariden, die ein terminales Galactose-(1->4)-Glucose-Disaccharid umfassen, durch vollständige Fermentation durch die Zellen.

## Revendications

1. Cellule hôte bactérienne capable de produire des oligosaccharides neutres ou fucosylés comprenant un disaccharide de galactose-(1->4)-glucose terminal, et comprenant
(i) au moins une séquence d'acide nucléique recombinant codant pour une protéine comportant une activité de bêta-1,4-galactosyltransférase et étant capable de galactosyler un monosaccharide de glucose libre pour générer de manière intracellulaire du lactose,
(ii) au moins une séquence d'acide nucléique recombinant codant pour une glycosyltransférase qui est choisie parmi au moins l'une des suivantes : une fucosyltransférase, une sialyltransférase, une glucosamyltransférase, une galactosyltransférase,
(iii) une modification génétique qui conduit à une surexpression de l'UDP-galactose-4'-épimérase ou de l'UDP-galactose-4'-épimérase en combinaison avec la glucose-1-phosphate-1-uridylyltransférase pour générer de manière intracellulaire de l'UDP-galactose,
(iv) au moins une séquence d'acide nucléique recombinant codant pour une protéine comportant une activité d'invertase et capable de convertir le saccharose pour générer de manière intracellulaire du fructose et du glucose,
et laquelle cellule hôte bactérienne est capable de générer l'oligosaccharide sans l'ajout exogène de lactose, glucose et fructose.

2. Cellule hôte bactérienne de la revendication 1, dans laquelle la glycosyltransférase est choisie parmi au moins l'une des suivantes : l'alpha-1,2-fucosyltransférase, l'alpha-1,3-fucosyltransférase, la bêta-1,3-N-acétyl-glucosamyltransférase, la bêta-1,3-galactosyltransférase, la bêta-1,4-galactosyltransférase, l'alpha-2,6-sialyltransférase, l'alpha-2,3-sialyltransférase, la bêta-1,6-galactosyltransférase, ou un fragment fonctionnel de celles-ci.

3. Cellule hôte bactérienne de l'une des revendications 1 ou 2, comprenant en outre une séquence d'acide nucléique codant pour au moins l'une des protéines supplémentaires suivantes :
une protéine exportatrice ou une perméase exportant l'oligosaccharide synthétisé à partir de la cellule hôte.

4. Cellule hôte bactérienne de la revendication 3, dans laquelle la protéine exportatrice est le transporteur d'efflux de sucre codé par le gène *yberc0001_9420* de *Yersinia bercovieri* ATCC 43970 ou un fragment fonctionnel de celui-ci.

5. Cellule hôte bactérienne de l'une quelconque des revendications 1 à 4, comprenant en outre une séquence d'acide nucléique codant pour au moins l'une des protéines supplémentaires suivantes : une fructokinase, des protéines impliquées dans la biosynthèse du GDP-fucose, du GDP-mannose, de l'UDP-glucose, de l'UDP-N-acétylglucosamine, de l'UDP-galactose, de l'UDP-N-acétylgalactosamine, de l'acide UDP-glucuronique, de l'acide CMP-sialique.

6. Cellule hôte bactérienne de l'une des revendications 4 ou 5, dans laquelle l'acide nucléique est un acide nucléique endogène ou recombinant.

7. Cellule hôte bactérienne de l'une quelconque des revendications 4 à 6, dans laquelle l'acide nucléique est homologue ou hétérologue.

8. Cellule hôte bactérienne de l'une quelconque des revendications précédentes comprenant en outre des séquences de contrôle permettant la surexpression contrôlée de séquences d'acides nucléiques endogènes ou recombinants.

9. Cellule hôte bactérienne de l'une quelconque des revendications précédentes, dans laquelle la bêta-1,4-galactosyltransférase est codée par un gène choisi dans le groupe constitué par *IgtB* de *Neisseria meningitidis* et *lex-1 d'Aggregatibacter aphrophilus NJ8700,* ou des fragments fonctionnels de ceux-ci.

10. Cellule hôte bactérienne de l'une quelconque des revendications précédentes, ladite cellule hôte bactérienne étant une souche d*'Escherichia coli,* une espèce de *Lactobacillus,* une souche de *Corynebacterium glutamicum,* ou une souche de Clostridium.

11. Cellule hôte bactérienne de l'une quelconque des revendications précédentes, comprenant en outre au moins l'un des gènes du groupe de gènes *E. coli W csc.*

12. Cellule hôte bactérienne de l'une quelconque des revendications précédentes, ladite cellule hôte bactérienne étant modifiée pour ne pas exprimer de protéines dégradant de manière intracellulaire des précurseurs dudit oligosaccharide.

13. Cellule hôte bactérienne de l'une quelconque des revendications précédentes, ladite cellule hôte bactérienne étant en outre génétiquement modifiée pour contenir une séquence d'acide nucléique codant pour une glycosidase pour la dégradation spécifique d'oligosaccharides interférents, tels que des intermédiaires, des produits secondaires ou des oligosaccharides endogènes générés par la cellule hôte bactérienne, ladite expression de ladite glycosidase étant sous le contrôle d'une séquence régulatrice.

14. Procédé pour la production d'au moins un oligosaccharide neutre ou fucosylé d'intérêt par fermentation totale à travers des cellules hôtes génétiquement modifiées, l'oligosaccharide d'intérêt comprenant un disaccharide de galactose-(1->4)-glucose terminal, et le procédé comprenant les étapes de :
a) obtention d'une cellule hôte bactérienne selon l'une quelconque des revendications précédentes ;
b) culture desdites cellules hôtes bactériennes de l'étape a), ladite culture étant réalisée (I) à partir d'une source de carbone qui est le saccharose, ladite cellule hôte étant capable de générer du glucose libre dans la cellule à partir de ladite source de carbone, et (II) sans apport externe de lactose pendant l'exécution du procédé, produisant ainsi ledit oligosaccharide d'intérêt.

15. Utilisation d'une cellule hôte bactérienne selon l'une quelconque des revendications 1 à 11 pour produire des oligosaccharides comprenant un disaccharide de galactose-(1->4)-glucose terminal par fermentation totale à travers lesdites cellules.
